# EUROPEAN PATENT APPLICATION

(11) **EP 2 589 667 A1**
(43) Date of publication of application: **08.05.2013**
(21) Application number: 11800939.8
(22) Date of filing: 30.06.2011
(51) Int. Cl.: C12Q 1/68, C12N 15/09

(54) **HIGHLY SENSITIVE METHOD FOR DETECTING MUTATED GENE**

(30) Priority: 30.06.2010 JP 2010148450
(71) Applicant: Mitsubishi Chemical Medience Corporation, Tokyo 108-8559 (JP)
(72) Inventor: MATSUMOTO, Hideo, Tokyo 108-8559 (JP); OHIDE, Akira, Tokyo 108-8559 (JP); MATSUDA, Koichiro, Tokyo 108-8559 (JP); FUJIMOTO, Hideya, Tokyo 108-8559 (JP)
(74) Representative: Vossius & Partner
(86) International application number: PCT/JP2011/065006
(87) International publication number: WO 2012/002477

(57) **Abstract**

Various highly sensitive detection methods, particularly improved PNA-LNA-PCR clamp methods, are provided as methods for detecting the presence or absence of a mutated gene contained in a gene pool rapidly, in a simple manner, with high accuracy, and with high sensitivity. As a step before the main step for detection, a pre-amplification step comprising allowing (1) a clamp primer consisting of PNA which hybridizes with all or part of a target site having a sequence of a wild-type gene or a sequence complementary to the wild-type gene, (2) a primer capable of amplifying a region comprising a target site having a sequence of the mutated gene, and (3) the gene pool to coexist in a reaction solution for gene amplification, and selectively amplifying the region comprising a target site of the mutated gene by a gene amplification method.

SEQUENCE LISTING

<110>  Mitsubishi Chemical Medience Corporation

<120>  Highly sensitive method for detecting mutated gene

<130>  MCM-889

<150>  JP 2010-148450
<151>  2010-06-30

<160>  6

<170>  PatentIn version 3.5

<210>  1
<211>  20
<212>  DNA
<213>  Artificial Sequence

<220>
<223>  Description of Artificial Sequence: primer

<400>  1
gtgcatcgct ggtaacatcc                                                    20


<210>  2
<211>  20
<212>  DNA
<213>  Artificial Sequence

<220>
<223>  Description of Artificial Sequence: primer

<400>  2
tgaggttcag agccatggac                                                    20


<210>  3
<211>  20
<212>  DNA
<213>  Artificial Sequence

<220>
<223>  Description of Artificial Sequence: primer

<400>  3
gcatgaacta cttggaggac                                                    20


<210>  4
<211>  20
<212>  DNA
<213>  Artificial Sequence

<220>
<223>  Description of Artificial Sequence: primer

<400>  4
acctaaagcc acctccttac                                                    20


<210>  5
<211>  21

<212>  DNA
<213>  Artificial Sequence

<220>
<223>  Description of Artificial Sequence: primer

<400>  5
tgtcataggg actctggatc c                                                21


<210>  6
<211>  19
<212>  DNA
<213>  Artificial Sequence

<220>
<223>  Description of Artificial Sequence: primer

<400>  6
agcagaaact cacatcgag                                                   19

## Description

### TECHNICAL FIELD

The present invention relates to a method for detecting a known mutated gene which coexists with a group of wild-type genes.

### BACKGROUND ART

Cancer is one of the three major lifestyle diseases as well as cardiac diseases and cerebrovascular diseases, and is currently the biggest cause of death in Japan. Generation of cancer generally originates with the fact that part of cells constituting a human body is cancerated to become cancer cells. In many cases, canceration of cells is caused by the mutation of a gene that is involved in cell division, cell proliferation and the like. In many cases, such a mutation of a gene which causes cancer is a point mutation in which one base on the base sequence of the gene is replaced with another base.

Early detection of cancer cells is important for the treatment of cancer at an early stage. The presence or absence of cancer cells is generally diagnosed under a microscope by a cytotechnologist or the like, after collecting pathologic tissue slices or cells from a subject, and fixing and staining the tissue slices or cells. However, such a pathologic diagnostic method had problems that the method required specialistic knowledge and technique of structural morphology, several days were required for obtaining a result, and a massive treatment was difficult. Therefore, in recent years, methods for detecting cancer cells at a genetic level quickly and conveniently by applying a gene amplification method such as a PCR method have been developed. For example, mutated gene detection methods using a nucleic acid analogue such as PNA (Peptide Nucleic Acid) or LNA (Locked Nucleic Acid) as a primer or a probe may be exemplified.

PNA is a nucleic acid analogue which is artificially and chemically synthesized, which is also called as a peptide nucleic acid. PNA has a structure in which a basic backbone of a nucleic acid constituted by pentose and phosphoric acid is replaced with a polyamide backbone having no charge including glycine as units. PNA binds more specifically and strongly to a nucleic acid having a complementary nucleotide sequence in comparison with DNA and RNA. In contrast, since PNA is a chemically synthesized substance, it has a property that nucleic acid polymerases and nucleases do not act thereon. By utilizing such a property, a method for selectively amplifying only a mutated gene by suppressing a wild-type gene by performing a PCR method using a PNA oligomer that is specific to a wild-type ras gene is known, as described in non-patent literature 1.

LNA is a nucleic acid analogue also called as a locked nucleic acid (patent literature 1), and is an inclusive term of molecules having a structure that is linked by the methylene bond between the 2'-oxygen and 4'-carbon of the ribose ring of a ribonucleoside. Similarly to PNA, LNA has high affinity for a nucleic acid having a complementary nucleotide sequence, and binds thereto more specifically and strongly in comparison with DNA and RNA. In contrast, unlike PNA, LNA has sensitivity against nucleases, and may also act as a primer for nucleic acid polymerases. By utilizing such properties, a method for detecting a Factor V Leiden mutation that increases the incidence of vein thromboembolism using a PCR method with an LNA probe was reported, as described in non-patent literature 2.

However, the majority of cell populations that are obtained from a subject are generally normal cells having wild-type genes, and only negligible cancer cells having a mutated gene are contained. Therefore, even if a mutated gene detection method utilizing the above-mentioned gene amplification method was conducted, the detection sensitivity was low due to the high background of the wild-type genes, and the reliability of the result was not sufficient. Therefore, many problems still remained for actually using the method as a gene testing in clinical practice. However, such technique for detecting a mutated gene from many wild-type genes has an extremely important meaning not only in an early detection of cancer but also in clinical therapies for cancer. Significant examples thereof are the sensitivities of molecular target drugs Gefitinib (product name "Iressa" manufactured and distributed by AstraZeneca) and Erlotinib (product name "Tarceva" manufactured and distributed by Chugai Pharmaceutical Co., Ltd.) against non-small cell lung cancer (hereinafter referred to as "NSCLC").

NSCLS is a disease that accounts for about 75% of the cases of lung cancer. Since the cancer progresses quickly, more than half of patients are inoperable at the time of detection of the cancer in many cases, and the mortality rate is high. For the progressive lung cancer, a treatment using a chemotherapy using a platinum preparation or Docetaxel as an anticancer drugs and a radiotherapy in combination is generally conducted, but a sufficient effect has not been achieved except for some cases (non-patent literature 3).

The actions of conventional anticancer drugs widely inhibit DNA synthesis and cell division, whereas Gefitinib and Erlotinib target human epidermal growth factor receptor (hereinafter referred to as EGFR). EGFR forms a dimer when a ligand such as epidermal growth factor (EGF) binds to the extracellular domain of EGFR, thereby causes tyrosine kinase activation of the intracellular domain. By this activity, autophosphorylation is caused, and an intercellular signal-transducing pathway at the downstream is operated. From the results that the activation of EGFR is involved in cell proliferation, that mutations of the EGFR gene were confirmed in many NSCLC patients, that overexpression of the EGFR protein is found in NSCLC patients, and the like, it is considered that the mutations of the EGFR gene is a cause of the onset of NSCLC. Gefitinib is called as a molecular target drug since it has an action of blocking signal transduction by inhibiting the autophosphorylation of EGFR.

Gefitinib was approved in 2002 in Japan. A dramatic therapeutic effect appeared in some NSCLC patients by administering the therapeutic drug. On the other hand, cases of death caused by its side effects such as interstitial pneumonia were generated. Although the cause of such bilateral characters of action and effect of Gefitinib has not been clarified yet, in recent years, a close relationship between a gene mutation of EGFR and the effectiveness of Gefitinib was reported in non-patent literatures 4 and 5. According to these documents, all of the NSCLC patients who could obtain a fine therapeutic effect by the administration of Gefitinib (Gefitinib-sensitive patients) had a mutation at a specific position on the EGFR gene. Therefore, it was suggested that Gefitinib acts as not an activity inhibitor for EGFR but an activity inhibitor for mutated EGFR. Therefore, if the presence or absence of a specific mutation on the EGFR gene can be detected, it becomes possible to decide whether or not Gefitinib may be administered to NSCLC patients.

As in the above-mentioned cases, the detection of a mutated gene which coexists with a group of wild-type genes is extremely important for realizing an early detection of cancer and tailor-made therapies that correspond to individual patients, and development of a method capable of detecting such a mutated gene conveniently and with high sensitivity is desired.
As such a method, a "PNA-LNA-PCR clamp method" (patent literature 2) capable of detecting the presence or absence of a mutated gene contained in a gene pool rapidly and conveniently, with high accuracy and high sensitivity, by performing a gene amplification reaction of a target site using a clamp primer consisting of PNA having the sequence of a wild-type gene and a mutation probe containing LNA and consisting of the sequence of a mutated gene (patent literature 2) is known.
According to the PNA-LNA-PCR clamp method, a detection is possible even if the content of the mutated gene in the gene pool is 0.1%, and emergence of a background associated with the amplification reaction is hardly detected. Furthermore, the time required for obtaining the result is only about 2 hours.
Furthermore, a point mutation in exon 20 is also known, and a PNA-LNA-PCR clamp method for detecting the mutation was reported, as described in non-patent literature 6.

### CITATION LIST

### PATENT LITERATURE

[Patent literature 1] Japanese Translation Publication (Kohyo) No. 2002-521310
[Patent literature 2] Japanese Patent No. 4216266 NON-PATENT LITERATURE

[Non-patent literature 1] Thiede C. et al., Nucleic Acids Res., 1996, 24, 983-984
[Non-patent literature 2] Orum H. et al., Clin Chem., 1999, 45:11; 1898-1905
[Non-patent literature 3] Muhsin M. et al., Nat.Rev.Drug Discov., 2003, 2(7); 515-516.
[Non-patent literature 4] Paez G.J. et al., Science, 2004, 304; 1497-1500
[Non-patent literature 5] Lynch T.J. et al., N Engl J Med, 2004, 350; 2129-2139
[Non-patent literature 6] Miyazawa H. et al., Cancer Sci., 2008, 99; 595-600

### SUMMARY OF INVENTION

### TECHNICAL PROBLEM

In a specimen which can be collected relatively easily from a patient, the ratio of tumor cells is decreased due to the incorporation of peripheral blood or normal cells in many cases. For example, it is expected that peripheral blood containing many normal cells is incorporated into a pleural effusion specimen to decrease the content of tumor cells, thereby the content of a mutated gene that is considered to be included in the tumor cells is decreased. Similarly, the same problem occurs in a bronchoalveolar lavage fluid since normal cells which have been scraped by a brush at the time of collection are incorporated into the specimen. Such specimens in which the content of tumor cells is readily decreased are not sufficient in view of detection sensitivity and reliability, and a method by which a detection can be conducted with higher sensitivity is desired. Examples described in patent literature 2 indicate that 0.1% of a mutated gene can be detected. However, these Examples are results obtained in a gene pool containing a plasmid in which only a specific region of the EGFR gene was cloned. In a gene pool obtained from a clinically-used specimen, human genomic DNA other than the EGFR gene accounts for a majority, and the content of the EGFR gene in the gene pool containing various genes is lower than that in the gene pool including the plasmid. Therefore, the detection sensitivity of the EGFR mutated gene is decreased in some cases. Furthermore, it is highly possible that a gene pool obtained from a clinical specimen includes human genomic DNA derived from normal cells (including the normal EGFR gene), and thus a detection of the mutated gene with higher sensitivity is required. The present inventors have invented a method for detecting a mutated gene with higher sensitivity than prior art by conducting a pre-amplification step (preliminary amplification) using a PNA clamp method as a gene pool to be subjected to conventional highly-sensitive detection methods such as a PNA-LNA-PCR clamp method, thereby increasing the content of the mutated gene and detecting the mutated gene with high sensitivity using a PNA-LNA-PCR clamp method as a later step.

Therefore, an object of the present invention is to provide an improved method of various high-sensitivity detection methods, particularly a PNA-LNA-PCR clamp method, as a method capable of detecting the presence or absence of a mutated gene contained in a gene pool rapidly and conveniently, with high accuracy and high sensitivity.

### SOLUTION TO PROBLEM

The object may be solved by the following present invention:
[1] A method for detecting the presence or absence of a known mutated gene contained in a gene pool, said method comprising the steps of:
   (1) allowing
      (1a) a clamp primer consisting of PNA which hybridizes with all or part of a target site having a sequence of a wild-type gene or a sequence complementary to the wild-type gene,
      (1b) a primer capable of amplifying a region comprising a target site having a sequence of the mutated gene, and
      (1c) the gene pool
         to coexist in a reaction solution for gene amplification, and selectively amplifying the region comprising a target site of the mutated gene by a gene amplification method, and
   (2) selectively detecting a detection region comprising the target site of the mutated gene by a gene detection method, using an amplified product obtained in step (1) or part thereof as a template, to detect the presence or absence of the mutated gene.
[2] The method of [1], wherein step (2) is a step of:
   allowing
      (2a) a clamp primer consisting of PNA which hybridizes with all or part of a target site having a sequence of the wild-type gene, said clamp primer having a nucleotide sequence the same as or different from that of clamp primer (1a) used in step (1),
      (2b) a mutation probe which hybridizes with all or part of a target site having a sequence of the mutated gene, and at least part of which consists of LNA, and
      (2c) an amplified product obtained in step (1) or part thereof
   to coexist in a reaction solution for gene amplification, and selectively amplifying a detection region comprising the target site of the mutated gene by a gene amplification method, to detect the presence or absence of the mutated gene.
[3] The method of [1], wherein step (2) is a step of:
   allowing
      (2a') a clamp primer consisting of PNA which hybridizes with all or part of a target site having a sequence complementary to the wild-type gene, said clamp primer having a nucleotide sequence the same as or different from that of clamp primer (1a) used in step (1),
      (2b) a mutation probe which hybridizes with all or part of a target site having a sequence complementary to the mutated gene, and at least part of which consists of LNA, and
      (2c) an amplified product obtained in step (1) or part thereof
   to coexist in a reaction solution for gene amplification, and selectively amplifying a detection region comprising the target site of the mutated gene by a gene amplification method, to detect the presence or absence of the mutated gene.
[4] The method of any of [2] or [3], wherein the gene amplification method in step (2c) is a PCR method.
[5] The method of any of [2] to [4], wherein the mutation probe comprises RNA.
[6] The method of any of [2] to [5], wherein the mutation probe is labeled with a fluorescent substance at one terminus, and is labeled with a quencher which suppresses the fluorescent substance at the other terminus.
[7] The method of any of [2] to [5], wherein the amplified product of the detection region of the mutated gene is stained with a nucleic acid stain.
[8] The method of any of [2] to [7], wherein an increase of the amplified product of the detection region of the mutated gene is sequentially detected by an increase of fluorescent intensity.
[9] The method of any of [2] to [8], wherein the mutation is a point mutation.
[10] The method of any of [2] to [8], wherein the mutation is a deletion mutation.
[11] The method of any of [2] to [10], wherein the clamp primer has a chain length of 14 to 18 nucleotides.
[12] The method of any of [1], wherein the gene detection method in step (2) is an invader method, a TaqMan-PCR method, a Scorpion ARMS method, a DNA chip method, a PCR-RFLP method, a PCR-SSCP method, a PCR-HRM method, a gFCS method, or a Southern blotting method.
[13] The method of any of [1] to [12], wherein the mutated gene is a mutated gene of a human epidermal growth factor receptor.

### ADVANTAGEOUS EFFECTS OF INVENTION

According to the present invention, the presence or absence of a mutated gene contained in a gene pool can be detected with high accuracy and high sensitivity, even if the detection is difficult even if using various known highly sensitive detection methods, particularly a PNA-LNA-PCR clamp method, like a specimen which is liable to include a reduced content of cancer cells, such as a pleural effusion specimen or a bronchoalveolar lavage fluid.

### BRIEF DESCRIPTION OF THE DRAWINGS

FIG. 1 is a drawing to explain the principle of embodiment 1.
FIG. 2 is a drawing to explain the principle of embodiment 5.

### DESCRIPTION OF EMBODIMENTS

The method of the present invention is an improved method of various known highly sensitive detection methods, in particular, PNA-LNA-PCR clamp methods (for example, the PNA-LNA-PCR clamp method described in Japanese Patent No. 4,216,266), and a specific pre-amplification step (preliminary amplification) is performed as a step before a highly sensitive detection method (main detection step). For example, when a PNA-LNA-PCR clamp method is used as the highly sensitive detection method, the present invention can be performed in a fashion similar to a conventional PNA-LNA-PCR clamp method, except that a specific pre-amplification step (preliminary amplification) is performed as a step before a selective amplification and detection step (hereinafter referred to as a main amplification step).
Hereinafter, after various highly sensitive detection methods to which the present invention can be applied are exemplified, a normal PNA-LNA-PCR clamp method as a preferable embodiment of the present invention will be explained based on embodiments 1 to 12, and then, the pre-amplification step in the method of the present invention will be explained.

The highly sensitive detection method to which the present invention can be applied is not limited, so long as it is a gene detection method capable of detecting a known mutated gene (particularly, a low content of mutated gene) which coexists with a group of wild-type genes. Examples of the methods include gene amplification methods such as a PNA-LNA-PCR clamp method, an invader method, a TaqMan-PCR method, and a Scorpion ARMS method, and gene identification methods such as a DNA chip method, a PCR-RFLP method, a PCR-SSCP method, a PCR-HRM method, a gFCS method, and a Southern blotting method.
Hereinafter the present invention will be explained based on an exemplified embodiment using a PNA-LNA-PCR clamp method as the highly sensitive detection method, but the present invention is not limited to the PNA-LNA-PCR clamp method.

### <Embodiment 1>

Embodiment 1 will be explained. This embodiment mainly relates to claim 2. In summary, this embodiment is a method for detecting the presence or absence of a known mutated gene contained in a gene pool, the method comprising the steps of allowing:
a clamp primer consisting of PNA which hybridizes with all or part of a target site having a sequence of the wild-type gene;
a mutation probe which hybridizes with all or part of a target site having a sequence of the mutated gene, and at least part of which consists of LNA; and
the gene pool;
to coexist in a reaction solution for gene amplification, and selectively amplifying a detection region comprising a target site of the mutated gene by a gene amplification method, to detect the presence or absence of the mutated gene.

Requirements of embodiment 1 will be explained. The definitions of the terms as used herein, such as DNA, RNA, nucleic acid, gene, gene expression, code, complementary, template, promoter, probe, primer, hybridization, and PCR, are the same as those currently and commonly used in molecular biology, genetics, genetic engineering, and the like.

The term "wild-type gene" as used herein means a gene which has no mutations and contains genetic information having its original normal functions. The term "genetic information" as used herein includes not only a transcriptional region which encodes information of mRNA, tRNA, rRNA, snRNA, and the like, but also a regulatory region such as a promoter which is required for gene expression.

The term "mutated gene" as used herein means a gene in which a mutation has occurred. The term "mutation" as used herein means a change in the sequence of a nucleic acid such as DNA and RNA, and includes a base substitution, insertion, deletion, inversion, duplication, translocation, and the like used in genetics and the like. The region of the mutation in a mutated gene is not limited to a transcriptional region, but includes a regulatory region such as a promoter which is required for gene expression. In this regard, the mutation in a mutated gene does not require a functional change.

The term "gene pool" as used herein means a gene population consisting of a number of genes, for example, genomic DNA obtained from whole cells contained in a patient's pleural effusion. The percentage of presence of the mutated gene in the target gene to be detected is not limited. For example, it may be 100% of a wild-type gene, or 50% of a wild-type gene and 50% of a mutated gene. Genes contained in a gene pool may be genomic DNA obtained from cells, DNAs obtained by a reverse-transcription reaction using mRNAs prepared from cells as a template, or an artificial mixture of a number of cloned genes.

The term "sequence" as used herein means a nucleotide sequence, i.e., a sequence of bases in a gene, a primer, or a probe. The nucleotide sequence may be composed of nucleic acids such as DNA and RNA, nucleic acid analogues such as PNA and LNA, or a combination thereof. For example, a nucleotide sequence consisting of DNA is designated "DNA sequence", and a nucleotide sequence consisting of PNA is designated "PNA sequence". The term "polynucleotide" as used herein means a compound consisting of DNA, RNA, PNA, or LNA, or any combination thereof.

The term "target site" as used herein means a site in which a mutated base exists or a base is deleted by a mutation in a mutated gene, and a site to be detected as a target in the present invention, including a wild-type gene. For example, in the case of a base substitution, the target site is a base which is substituted in both a wild-type gene and a mutated gene. In the case of an insertion, the target site in a mutated gene is an inserted base, and the target site in a wild-type gene is a site into which the base is inserted in the mutated gene. In the case of a deletion, the target site in a mutated gene is a site in which a base is deleted by the deletion, and the target site in a wild-type gene is the deleted base in the mutated gene. The sequence of a target site may be a side having a sequence which encodes genetic information (hereinafter referred to as a sense side or sequence), or a side having a sequence complementary to the sense sequence (hereinafter referred to as an antisense side or sequence).

The term "PNA" as used herein includes not only conventional PNA, but also a PNA derivative which is an improvement based on PNA and has properties similar to PNA, for example, gripNA provided by Active Motif.

The term "clamp primer" as used herein means a primer consisting of PNA which hybridizes with a target site, regardless of whether it is used in the main amplification step of a PNA-LNA-PCR clamp method or the pre-amplification step described in detail below. The clamp primer is composed of PNA over the entire sequence. Therefore, when it hybridizes with a nucleotide sequence as a primer, it shows an extremely high selectivity to the nucleotide sequence due to the properties of PNA. Further, since PNA is not affected by nucleic acid polymerases and nucleases, the clamp primer does not function as a primer and is completely resistant to nucleases. This embodiment is characterized by the fact that the nucleotide sequence of the clamp primer is a sequence complementary to that of a wild-type gene. The nucleotide sequence of the clamp primer is not limited, so long as it specifically hybridizes with part or all of a target site. The length of the clamp primer is not limited, so long as it is 10 to 25 nucleotides. Since the number of a wild-type gene is larger than that of a mutated gene in a gene pool, the concentration of the clamp primer is preferably 10 times or more that of the mutation probe in the reaction solution for gene amplification so that the clamp primer can sufficiently hybridize with the wild-type gene. For example, when the concentration of the mutation probe is 100 nmol/L, the concentration of the clamp primer is approximately 1 µmol/L, preferably approximately 5 µmol/L. The synthesis of a clamp primer may be outsourced to a supplier of PNA oligonucleotides, for example, Greiner Japan.

The term "LNA" as used herein includes not only conventional LNA variants such as oxy-LNA, thio-LNA, and amino-LNA, but also LNA derivatives which are improvements based on LNA and have properties similar to LNA.

The term "mutation probe" as used herein means a mutation probe which hybridizes with a target site, and at least one nucleotide contained in the DNA sequence of which is replaced with LNA. This embodiment is characterized by the fact that the nucleotide sequence of the mutation probe is a sequence complementary to that of the mutated gene. The nucleotide sequence of the mutation probe is not limited, so long as it specifically hybridizes with part or an entire of a target site, and its Tm value is within a range of preferably 50°C to 65°C, and more preferably 54°C to 56°C. It is preferable that the nucleotide sequence of the mutation probe is manually designed to confirm the Tm value using an LNA Tm prediction tool (http://lna-tm.com/). The number of nucleotides contained in the mutation probe is 10 to 50 nucleotides, preferably 15 to 25 nucleotides. The number of LNA is not limited, so long as it is one or more, and all the nucleotides may be replaced with LNA. With respect to a position at which a nucleotide is replace with LNA in the mutation probe, when there are one or more bases which are not found in a target region of a wild-type gene, it is preferable to replace at least one of these bases with LNA. For example, in the case that a mutation is an insertion of some bases, it is preferable to replace at least one of the inserted bases with LNA. The synthesis of a mutation probe may be outsourced to a supplier of LNA oligonucleotides, for example, Exicon (the Netherlands) or Integrated DNA Technologies (IDT, the United States).

The term "gene amplification method" as used herein means a method for amplifying a detection region comprising a target site using a primer for amplification. The gene amplification reaction used in this embodiment is not limited, so long as the detection region can be amplified. The gene amplification reaction may be, for example, a PCR method, an ICAN method (an isothermal and chimeric primer-initiated amplification of nucleic acids method), or a LAMP method (a Loop-mediated isothermal amplification method). A chemical reaction for gene amplification in the gene amplification method is designated "gene amplification reaction".

The term "reaction solution for gene amplification" as used herein means a solution containing reagents and the like which are required for the gene amplification reaction the gene amplification method. The formulation of the reaction solution for gene amplification needs minor modifications depending on the gene amplification method used, but the reaction solution for gene amplification basically contains 4 kinds of deoxynucleoside triphosphates (dATP, dTTP, dCTP, and dGTP: hereinafter collectively referred to as dNTPs) as substrates, a DNA polymerase as an enzyme, a magnesium ion as a cofactor for the enzyme, and amplification primers as primers for elongation, in a buffer. The concentrations of dNTPs may be selected within a range of 100 nmol/L to 400 nmol/L as a final concentration of each. A DNA polymerase having properties suitable for the gene amplification method used may be used. For example, a strand displacement-type DNA polymerase is preferable for the LAMP method. The concentration of the magnesium ion may be selected within a range of 1 mmol/L to 6 mmol/L as a final concentration.

The "amplification primer" mentioned above is a primer which is used for amplifying a detection region in the gene amplification method. The amplification primer is composed of a nucleic acid such as DNA or RNA, LNA, or a combination thereof. The concentration of the amplification primer may be appropriately selected within a range of 100 nmol/L to 1 µmol/L as a final concentration.
In this case, it is preferable that an amplification primer which hybridizes with the upstream (i.e., 5' side) of the mutation probe is absent. Further, the sequence of the primer may contain a target site in this case. The number of amplification primers is not limited, so long as a detection region comprising a target site can be amplified by the gene amplification method. For example, when a single primer alone can hybridize with a template so that the directions of elongation are opposite to each other, the number of the amplification primers may be one. When primers can hybridize with a template so that the directions of elongation are opposite to each other, the primers may be a pair of different amplification primers. The number of nucleotides contained in the amplification primer is not limited, so long as it is within a range of 10 to 40 nucleotides, preferably 15 to 30 nucleotides, and more preferably 18 to 25 nucleotides. The distance between the amplification primers, i.e., the detection region, is not limited, so long as it is within a range of 100 nucleotides to 5,000 nucleotides (5 kb: hereinafter 1,000 nucleotides are represented by 1 kb), preferably 150 nucleotides to 2 kb. The sequence of the amplification primer is not limited, so long as a detection region comprising a target site can be amplified by the gene amplification method and its Tm value is within a range of 50°C to 65°C, preferably 55°C or 60°C. The sequence of the amplification primer may be designed manually or using an appropriate software for primer design. For example, Primer3 software (http://frodo.wi.mit.edu/cgi-bin/primer3/primer3.cgi) may be used. The mutation probe mentioned above may function as the amplification primer.
The buffer has an optimum pH and an optimum salt concentration capable of obtaining the activity of the DNA polymerase. Depending on the type of the gene amplification method used, the buffer may further contain ribonuclease H (RNaseH), reverse transcriptase (RT), or the like. Various types of reaction solutions for gene amplification are commercially available for each gene amplification method, and one attached to such a commercially available kit may be used. When Taq DNA polymerase is used as the enzyme, 10 mmol/L Tris-HCL (pH 8.3)/50 mmol/L KCl/2 mmol/L MgCl₂/2.5U Taq DNA polymerase may be exemplified as a basic formulation of its reaction solution for gene amplification. The present invention is not limited to these conditions.

The term "detection region" as used herein means a region comprising a target site amplified by performing the gene amplification reaction using the amplification primer. The amplified product of the detection region is amplified as a fragment consisting of a DNA sequence, excluding a portion derived from the amplification primer. According to the present invention, a detection region derived from a mutated gene is selectively amplified. Therefore, the amplified product of the detection region may be used in the detection of an amplified nucleic acid described below.

The term "coexist" as used herein means a state in which components coexist with each other in a reaction solution for gene amplification and can affect each other.

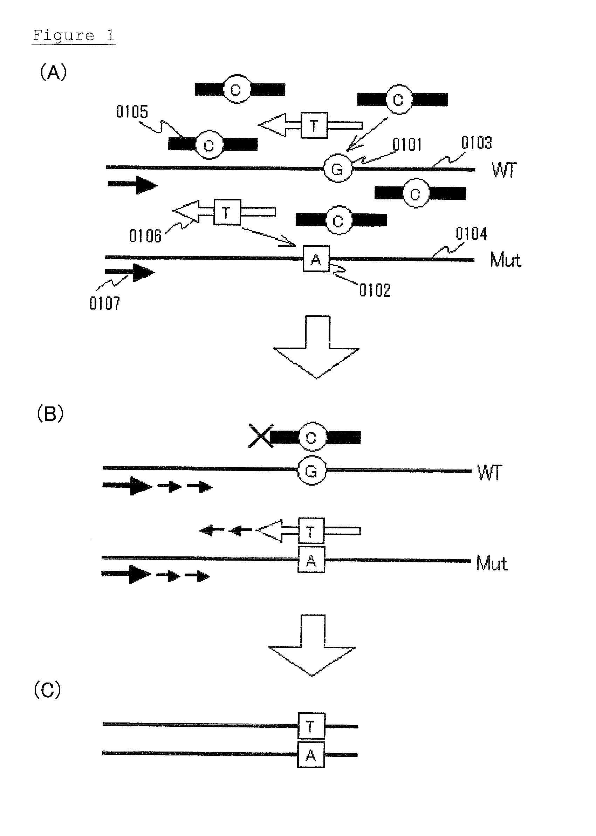

An example of the principle of a PNA-LNA-PCR clamp method is shown in FIG. 1, and will be explained hereinafter. The mutation of the mutated gene is a point mutation and the gene amplification reaction is a PCR method in the example of FIG. 1.

### (Hybridization of probe and primers to templates)

First, a clamp primer (0105) consisting of PNA having a sequence complementary to a target site (0101) of a wild-type gene (0103), and a mutation probe (0106) having a sequence complementary to a target site (0102) of a mutated gene (0104) and consisting of DNA containing LNA are allowed to coexist with an amplification primer (0107) and a gene pool in a reaction solution for gene amplification. Since wild-type genes are generally present in a gene pool in large amounts, the clamp primer is added in slight excess amount. As a result, the clamp primer, the mutation probe, and the amplification primer hybridize with the respective corresponding complementary sequences on the genes to be detected. All or parts of the nucleotide sequences of the clamp primer and the mutation probe accord with each other, because their target sites are the same. Therefore, both the hybridizations to the target sites essentially compete with each other. However, since PNA and LNA exhibit a high specificity to each complementary nucleotide sequence, even if the mutation is a difference in only one nucleotide, almost all the clamp primer hybridizes with the target site on the wild-type gene, and almost all the mutation probe hybridizes with the target site on the mutated gene, as shown in FIG. 1(A).

### (Primer elongation reaction)

Next, the resulting reaction solution for gene amplification is subjected to a primer elongation reaction using a gene amplification method. Since the clamp primer does not function as a primer, no elongation reaction occur, as shown in FIG. 1(B). Therefore, the amplification of the detection region comprising the target site of the wild-type gene is suppressed by the hybridization of the clamp primer. In contrast, LNA does not exhibit an inhibitory activity to primer elongation like PNA. Since the mutation probe can function as a primer, an elongation reaction occurs, and the detection region comprising the target site of the mutated gene is amplified between the mutation probe and the amplification primer, as shown in FIG. 1(c).

### (Gene amplification reaction cycle)

After the completion of the elongation reaction, the clamp primer, the mutation probe, and the amplification primer are again hybridized with the corresponding complementary sequences on the genes to be detected. For example, in the case using a PCR method, the reaction of the re-hybridization includes a thermal denaturation treatment by heating up to around 90°C, but this treatment is not necessarily needed, depending on the type of the gene amplification method. Examples of this include an ICAN method and a LAMP method in which amplification can be performed at a constant temperature of 50°C to 65°C. The cycles of gene amplification reaction are repeated by a gene amplification method to selectively amplify the detection region comprising the target site of the mutated gene. The number of the cycles varies in accordance with the gene amplification method or the type of a DNA polymerase used, and is not limited, so long as it is within a range of 30 to 55 cycles. It is preferably 35 to 50 cycles, and more preferably 40 to 45 cycles.

### (Detection of amplified product)

The detection region which is derived from the mutated gene and amplified by the gene amplification method is detected. The detection method is not limited, so long as the amplification of the detection region of the mutated gene can be confirmed. For example, the solution after the completion of the gene amplification reaction may be deproteinized using a phenol/chloroform (1:1) solution, and the resulting aqueous layer may be used directly, or after an additional purification by ethanol precipitation or using an appropriate purification kit, to measure the absorbance at a wavelength of 260 nm using a spectrophotometer; the amplified product may be electrophoresed using an agarose gel or a polyacrylamide gel, and may be detected by Southern blotting using an appropriate probe; it may be detected by a chromatography hybridization method using gold nanoparticles; or the turbidity of the solution caused by the amplified gene may be measured. Alternatively, the 5' terminus of the amplification primer may have been previously labeled with a fluorescent substance, and after the completion of the gene amplification reaction, the amplified product may be electrophoresed using an agarose gel or a polyacrylamide gel, and fluorescent emission may be captured on an imaging plate and may be detected using an appropriate detector. In these detection methods, more highly reliable results can be obtained by comparing the measured values with the results of an appropriate control. Further, more accurate results can be obtained by confirming the presence of the target site of the mutated gene in the amplified product by sequencing.

### <Embodiment 2>

Embodiment 2 will be explained. This embodiment mainly relates to claim 3. In summary, this embodiment is a method for detecting the presence or absence of a known mutated gene contained in a gene pool, the method comprising the steps of allowing:
a clamp primer consisting of PNA which hybridizes with all or part of a target site having a sequence complementary to the wild-type gene;
a mutation probe which hybridizes with all or part of a target site having a sequence complementary to the mutated gene, and at least part of which consists of LNA; and
the gene pool;
to coexist in a reaction solution for gene amplification, and selectively amplifying a detection region comprising the target site of the mutated gene by a gene amplification method, to detect the presence or absence of the mutated gene.

Whereas embodiment 1 is a method for detecting a mutated gene by using the sense sequence of a gene to be detected as a template, embodiment 2 is a method for detecting a mutated gene by using the antisense sequence of a gene to be detected as a template. Therefore, this embodiment is characterized in that the nucleotide sequence of the clamp primer is the same sequence as the sense sequence of a wild-type gene. Further, this embodiment is characterized in that the nucleotide sequence of the mutation probe is the same sequence as the sense sequence of a mutated gene. These features are based on the fact that the template which is used in the detection of a mutated gene of the present invention is not limited to the sense sequence, but the antisense sequence also may be used. Embodiment 2 is different from embodiment 1 in this point alone, and is the same as embodiment 1 with respect to other requirements and its principle, which will not be repeated herein.

Therefore, the sequence of the temperate may be the sense sequence or the antisense sequence in the present invention. However, with respect to the sequence of the clamp primer used in the present invention, a sequence at a side which contains fewer purine residues in the clamp primer sequence, or a sequence at a side which does not contain three or more consecutive guanidine residues in the clamp primer sequence is preferable. This is because an increase in the number of purine residues or three or more consecutive guanidine residues cause difficulty in the dissolution of PNA.

### <Embodiment 3>

Embodiment 3 will be explained. This embodiment mainly relates to claim 4. In summary, this embodiment is a method for detecting a mutated gene which is based on embodiment 1 or embodiment 2 as mentioned above and the gene amplification method of which is a PCR method. In the present embodiment, explanations for the same requirements and the like as those of embodiment 1 will not be repeated, but only requirements and the like characteristic of the present embodiment will be explained hereinafter.

The term "PCR method" as used herein includes not only an original PCR method based on the most fundamental principle, but also variations obtained by improving the original PCR method. Examples of the variations include a nested-PCR method and an RT-PCR method.

The conditions of the reaction solution for gene amplification in the present embodiment are the same as those in embodiment 1, and therefore, the explanation for the conditions will not be repeated, but methods characteristic of the present embodiment will be explained hereinafter.

The type of DNA polymerase used in the PCR method is not limited, so long as it has an activity of elongating an amplification primer. A heat-resistance DNA polymerase is preferable. For example, a Taq DNA polymerase or a Pfu DNA polymerase may be used, and various heat-resistance DNA polymerases developed by bioscience-related companies may be used.

With respect to cycle reactions, the PCR method may be a two-step PCR method consisting of thermal denaturation and annealing/elongation reaction, or a three-step PCR method consisting of thermal denaturation, annealing, and an elongation reaction.

In the case that the nested-PCR method is used, the conditions of the second gene amplification reaction are not limited, except that an amplification primer used in the second reaction (hereinafter referred to as inner amplification primer) is used instead of the amplification primer used in the first reaction (hereinafter referred to as outer amplification primer), and except that a dilution obtained by diluting a reaction solution for gene amplification after the completion of the first gene amplification reaction is used as the template, so long as a non-specific amplification of an amplified product can be suppressed. For example, the conditions of the second reaction may be the same as those of the first reaction, or the conditions may be appropriately altered, for example, by increasing or decreasing the number of reaction cycles based on the amount of the amplified product or the like. A pair of inner amplification primers is not limited, so long as at least one sequence is one or more nucleotides downstream (i.e., at the 3' side) of the sequence of the corresponding outer amplification primer. For example, the sequence of the other inner amplification primer may be the same as that of the corresponding outer amplification primer, or all of the inner amplification primers may almost overlap with the corresponding outer amplification primers. The dilution of the reaction solution for gene amplification after the completion of the first gene amplification reaction may be appropriately selected within a range of 1/10⁷ dilution to 1/10⁵ dilution, preferably a range of 5/10⁷ dilution to 5/10⁶ dilution.

Since the PCR method is the most commonly used gene amplification method, not only various reagents, equipments, and the like which are optimized for the method are easily available, but also there are a number of innovative technologies based on the method, and therefore, the method is very versatile. In common research laboratories and clinical laboratories for molecular biology or genetic engineering, reagents and equipments required for the PCR method are already provided in general, and it is convenient from the viewpoint that it is not necessary to purchase new reagents and equipments.

### <Embodiment 4>

Embodiment 4 mainly relates to claim 5. In summary, this embodiment is a method for detecting a mutated gene characterized in that it is based on any one of embodiments 1 to 3 and the mutation probe comprises RNA. In the present embodiment, explanations for the same requirements and the like as those of any one of embodiments 1 to 3 will not be repeated, but only requirements and the like characteristic of the present embodiment will be explained hereinafter.

In this embodiment, part of the mutation probe comprises RNA. Due to this feature, for example, when the mutation probe hybridizes with the amplified product in embodiment 5 described below, and the formed hybrid is treated with RNaseH, the RNA is digested and the fluorescent substance is separated from the quencher, and as a result, the suppression of the fluorescent substance is released to emit a fluorescence. It is convenient that the mutated gene can be detected by measuring the generated fluorescence.

### <Embodiment 5>

Embodiment 5 will be explained. Embodiment 5 mainly relates to claim 6. In summary, this embodiment is a method for detecting a mutated gene in which it is based on any one of embodiments 1 to 4 and the mutation probe is labeled with a fluorescent substance at one terminus, and it is labeled with a quencher which suppresses the fluorescent substance at the other terminus. In the present embodiment, explanations for the same requirements and the like as those of any one of embodiments 1 to 4 will not be repeated, but only requirements and the like characteristic of the present embodiment will be explained hereinafter.

The term "fluorescent substance" as used herein means a substance having the property that it becomes an excited state by absorbing an excitation light, and fluorescence is emitted when it returns to the ground state of the original. The fluorescent substance to be used for labeling is not limited, so long as the termini of the mutation probe can be labeled. Examples of the fluorescent substance include FAM, TET, HEX, Cy3, Cy5, Texas Red, TAMRA, and FITC.

The term "quencher" as used herein means a substance or like capable of absorbing the excitation energy of a fluorescent substance. The type used may be, for example, BHQ1, BHQ2, or Dabcyl. In this regard, since the range of fluorescence suppression is different depending on the type of substance to be suppressed, it is necessary to select a quencher which can suppress the emission of the fluorescent substance which forms a pair with the quencher on the mutation probe. The quencher is not limited within the range capable of suppressing the emission. For example, when the fluorescent substance is FAM or TET, BHQ1 in which the wavelength of the suppression range is slightly closer to a shortwave side (480 nm to 580 nm) may be selected. When the fluorescent substance is Cy3 or Texas red, BHQ2 in which the wavelength of the suppression range is slightly closer to a longwave side (550 nm to 650 nm) may be selected.

The term "terminus" as used herein means the 5' terminus or the 3' terminus of the nucleotide sequence of the mutation probe.

The term "labeled" as used herein means a state that the fluorescent substance and the quencher are added to the termini of the mutation probe by a chemical bond. In other words, one of the fluorescent substance and the quencher is chemically bound to the 5' terminus, and the other is chemically bound to the 3' terminus. The fluorescent substance is bound to the 5' terminus in many cases, but the fluorescent substance may be bound to the 3' terminus, so long as the same effects can be obtained. In addition, when the quencher or the fluorescent substance is bound to the 3' terminus, the mutation probe does not function as the amplification primer. This is because the quencher or the fluorescent substance inhibits the elongation reaction. Therefore, in this case, at least one amplification primer which hybridizes with the sequence at the 5' side (i.e., the upstream) of the mutation probe is required. Further, it is preferable that the DNA polymerase used in the gene amplification method has a 5'→3' exonuclease activity so that the mutation probe which is hybridizing with the target site does not avoid the elongation of the amplification primer.

### (Detection method of embodiment 5)

It is preferable that the detection method in the present embodiment utilizes the fluorescence emitted from the fluorescent substance. However, in the mutation probe used in the present embodiment, when the fluorescent substance bound to one terminus of the mutation probe is irradiated with an excitation light under normal conditions, it does not emit the fluorescence due to the suppression by the quencher which is present at the other terminus. Therefore, it is necessary to release the suppression by the quencher in order to utilize the fluorescence in the present embodiment. The method for release the quencher is not limited. For example, in a case using a mutation probe labeled with a fluorescent substance at the 5' terminus, an amplification primer which hybridizes with the upstream of the mutation probe, and a DNA polymerase having a 5'→3' exonuclease activity may be added to a reaction solution for gene amplification, and a gene amplification may be performed by a PCR method. In this case, the mutation probe is digested by the 5'→3' exonuclease activity of the DNA polymerase, and the fluorescent substance is released from the mutation probe, and as a result, the suppression by the quencher is released. Alternatively, part of the mutation probe may be formed with RNA, and the suppression may be released by the activity of RNaseH, as described in embodiment 4.

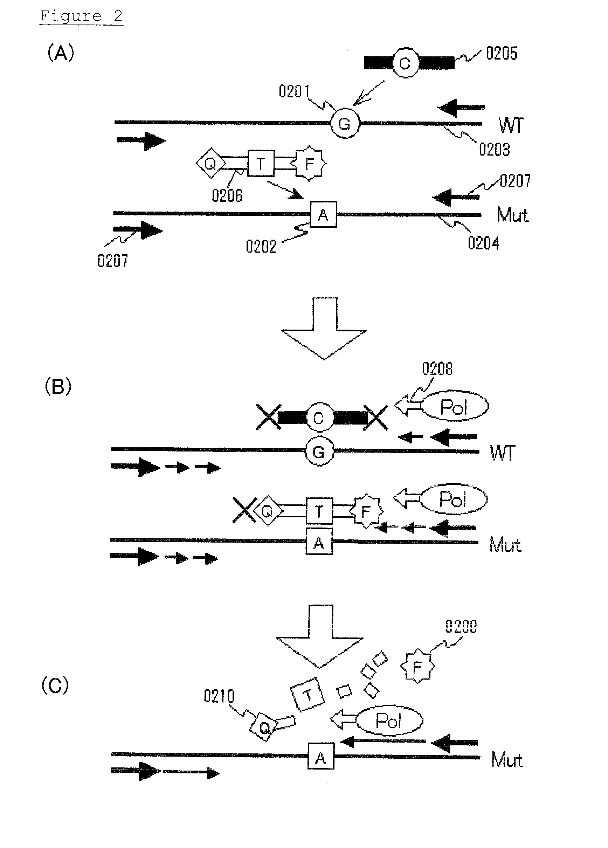

An example of the principle of the detection in the present embodiment is shown in FIG. 2, and will be explained hereinafter. The mutation of the mutated gene is a point mutation and the gene amplification reaction is a PCR method using a DNA polymerase having a 5'→3' exonuclease activity in the example of FIG. 2.

### (Hybridization of probe and primers to templates)

First, a clamp primer (0205) consisting of PNA having a sequence complementary to a target site (0201) of a wild-type gene (0203), and a mutation probe (0206) having a sequence complementary to a target site (0202) of a mutated gene (0204) and consisting of DNA containing LNA are allowed to coexist with two types of amplification primers (0207) and a gene pool in a reaction solution for gene amplification. The mutation probe is labeled with a fluorescent substance (0209) at the 5' terminus and a quencher (0210) at the 3' terminus. The quencher represses a fluorescence emission from the fluorescent substance under normal conditions. Similarly to the explanations of FIG. 1, due to a high specificity of PNA and LNA to each complementary sequence, even if the mutation is a difference in only one nucleotide, almost all the clamp primer hybridizes with the target site on the wild-type gene, and almost all the mutation probe hybridizes with the target site on the mutated gene, as shown in FIG. 2(A).

### (Primer elongation reaction)

Next, the resulting reaction solution for gene amplification is subjected to a primer elongation reaction using a gene amplification method. Since the clamp primer does not function as a primer, no elongation reaction occur, as shown in FIG. 2(B). Further, due to the nuclease resistance of PNA, even if a DNA polymerase used in the elongation reaction has a 5'→3' exonuclease activity (0208) like Taq DNA polymerase, the clamp primer is not digested by the DNA polymerase. Therefore, the amplification of a detection region comprising the target site of the wild-type gene is suppressed by the hybridization of the clamp primer, even if the two types of amplification primers which are designed so as to sandwich the clamp primer are allowed to coexist. In contrast, unlike PNA, LNA does not exhibit an inhibitory activity to primer elongation and resistance to nucleic acid nucleases. In this regard, the elongation reaction is inhibited because the quencher is bound to the 3' terminus of the mutation probe. Therefore, the elongation reaction occurs between the two types of amplification primers (0207) in this case. That is to say, the mutation primer is digested by the 5'→3' exonuclease activity of the DNA polymerase used in the elongation reaction, and the detection region comprising the target site on the mutated gene is amplified between the amplification primers. This digestion results in the release of the fluorescent substance, and the suppression by the quencher is released. The amplified mutated gene can be detected by detecting fluorescent substance emitted from the released fluorescent substance.

### <Embodiment 6>

Embodiment 6 will be explained. Embodiment 6 mainly relates to claim 7. In summary, the present embodiment is a method for detecting a mutated gene in which it is based on any one of embodiments 1 to 4 and an amplified product of a detection region of the mutated gene is stained with a nucleic acid stain. In the present embodiment, explanations for the same requirements and the like as those of any one of embodiments 1 to 3 will not be repeated, but only requirements and the like characteristic of the present embodiment will be explained hereinafter.

The term "nucleic acid stain" as used herein collectively refers to reagents with which nucleic acids or nucleic acid derivatives are stained by the binding thereto or the intercalation thereinto. Examples of the nucleic acid stain include an intercalator with which a nucleic acid is stained by the intercalation between bases of a doublestranded nucleic acid. Many types of intercalators are known, for example, ethidium bromide (EB), propidium iodide (PI), DAPI, acridine orange, various Hoechsts, various SYBRs, various SYNTOXs, various TOTOs, and the like. The type used is not limited, so long as the amplified product can be sufficiently stained.

The term "staining" as used herein means not only coloration of the amplified product which can be visually confirmed, but also fluorescent staining which can be confirmed by irradiating with an excitation light.

### (Detection method of embodiment 6)

Nucleic acid stains, including intercalators as described above, emit specific fluorescences when irradiated with an excitation light at a specific wavelength. Therefore, it is preferable that the detection method in the present embodiment utilizes this fluorescence emission. The method and the timing of the staining are not limited, so long as the staining is completed. For example, the staining may be performed by, after the completion of the gene amplification reaction, dipping the amplified product in a stain solution obtained by dissolving a nucleic acid stain, or the staining may be performed during the gene amplification reaction. The method for detection is not limited, so long as the amplified product stained can be detected. For example, the amplified product may be electrophoresed using an agarose gel or a polyacrylamide gel, the gel stained with the nucleic acid stain may be irradiated with an excitation light on a transilluminator, and a band at the position corresponding to the deduced size of the amplified product and a control band may be visually compared and confirmed. Alternatively, fluorescence emitted from the fluorescent substance may be detected using a light capture (an imaging device for chemiluminescence and fluorescence), and the amplified product may be detected by performing comparative determination of fluorescent intensity to a control using an analyzing software.

### <Embodiment 7>

Embodiment 7 will be explained. Embodiment 7 mainly relates to claim 8. In summary, the present embodiment is a method for detecting a mutated gene in which it is based on any one of embodiments 1 to 6 and an increase of an amplified product of a detection region of the mutated gene is sequentially detected by an increase in fluorescent intensity. In the present embodiment, explanations for the same requirements and the like as those of any one of embodiments 1 to 6 will not be repeated, but only requirements and the like characteristic of the present embodiment will be explained hereinafter.

The term "continuously" as used herein means "continuous data collection or measurement". Therefore, "an increase of the amplified product is continuously detected" means that the data as to an increase of the amplified product are continuously collected or measured in real time during the gene amplification reaction.

The detection is performed by "an increase in fluorescent intensity". The term "fluorescence" means a fluorescence or the like emitted from the fluorescent substance mentioned in embodiment 5 or the nucleic acid stain mentioned in embodiment 6 when the excited one returns to the ground state.

### (Detection method of embodiment 7)

The detection method of the present embodiment is not limited, so long as an increase in fluorescent intensity which reflects an increase in the amplified product can be sequentially detected. For example, a fluorescence generated by digesting a mutation probe the 5' terminus of which is labeled with a fluorescent substance by a DNA polymerase having a 5'→3' exonuclease activity to release the 5' terminus may be detected in real time (TaqMan probe method), as described in embodiment 5. Alternatively, a fluorescence generated by digesting an RNA portion of a mutation probe a terminus of which is labeled with a fluorescent substance by the addition of RNaseH may be detected in real time (cycling probe method). Alternatively, as described in embodiment 6, a nucleic acid stain such as SYBER Green I is added to the reaction solution for gene amplification to perform the gene amplification reaction while staining. An increase in the amplification product may be detected by measuring fluorescent intensity emitted from the intercalated nucleic acid stain while irradiating an excitation light.

### <Embodiment 8>

Embodiment 8 will be explained. Embodiment 8 mainly relates to claim 9. In summary, this embodiment is a method for detecting a mutated gene in which it is based on any one of embodiments 1 to 7 and the mutation is a point mutation. In the present embodiment, explanations for the same requirements and the like as those of any one of embodiments 1 to 7 will not be repeated, but only requirements and the like characteristic of the present embodiment will be explained hereinafter.

The term "point mutation" as used herein includes not only a mutation in which a nucleotide is replaced with a different nucleotide in the nucleotide sequence of a gene to be detected, but also a mutation in which a nucleotide is inserted into the nucleotide sequence. Therefore a site in which a nucleotide is replaced or inserted is the target site in the point mutation.

The mutation probe used in the present embodiment is characterized in that the target site has the sequence of the mutated gene and at least the target site is replaced with LNA. For example, in the case that the mutation is a substitution of a nucleotide and it is based on embodiment 1, the sequence of the mutation probe is a sequence complementary to the mutated gene, and the sequence of at least the substituted nucleotide (a nucleotide) is replaced with LNA. This enables the mutation probe to specifically hybridize with the mutated gene, even when the difference between the wild-type gene and the mutated gene is only a nucleotide. Further, the clamp primer having the sequence of the wild-type gene becomes difficult to hybridize with the mutated gene, and as a result, the sequence specificities of the mutated gene and the clamp primer to each target site become clear.

### <Embodiment 9>

Embodiment 9 will be explained. Embodiment 9 mainly relates to claim 10. In summary, this embodiment is a method for detecting a mutated gene in which it is based on any one of embodiments 1 to 7 and the mutation is a deletion mutation. In the present embodiment, explanations for the same requirements and the like as those of any one of embodiments 1 to 7 will not be repeated, but only requirements and the like characteristic of the present embodiment will be explained hereinafter.

The term "deletion mutation" as used herein means a mutation in which a nucleotide or the continuity of two or more nucleotides are deleted or lost from the nucleotide sequence of a wild-type gene, with respected to the gene to be detected. Therefore, it is not limited to the deletion commonly used in genetics. For example, it includes the case in which a gene to be detected is divided by chromosomal translocation or inversion, retroviral insertion, or the like. Therefore, in such a deletion mutation, a site at which a nucleotide or the continuity of two or more nucleotides are deleted or lost from the nucleotide sequence of a wild-type gene is the target site.

In the mutation probe used in the present embodiment, the target site has the sequence of the mutated gene and at least one nucleotide of the sequence of the mutation probe is replaced with LNA. In particular, when a specific nucleotide sequence which is not found in the wild-type gene is present in the target site, it is preferable that at least one nucleotide of the specific nucleotide sequence is replaced with LNA. For example, in the case in which the deletion mutation is an insertion of three nucleotides, it is preferable that at least one nucleotide of the three inserted nucleotides is replaced with LNA. The mutation probe may have all or part of the specific nucleotide sequence. When no nucleotides which are found in the wild-type gene are present at the target site, that is, in a case of the deletion commonly used in genetics, it is preferable that at least one nucleotide of the sequence of the mutation probe is replaced with LNA. It is most preferable that one of the nucleotides at the connected site newly generated by the result of deletion is replaced with LNA. According to the present embodiment, the mutation probe can hybridize with the mutated gene more specifically and more strongly, even when the mutation is a deletion mutation.

### <Embodiment 10>

Embodiment 10 will be explained. Embodiment 10 mainly relates to claim 11. In summary, this embodiment is a method for detecting a mutated gene according to any one of claims 2 to 10, in which it is based on any one of embodiments 1 to 9 and the clamp primer has a chain length of 14 to 18 nucleotides. In the present embodiment, explanations for the same requirements and the like as those of any one of embodiments 1 to 9 will not be repeated, but only requirements and the like characteristic of the present embodiment will be explained hereinafter.

The present embodiment is characterized in that the clamp primer is composed of 14 to 18 nucleotides. In the clamp primer consisting of PNA, a clamp primer of 13 nucleotides or less sometimes does not exhibit a sufficient clamping effect, and a clamp primer of 19 nucleotides sometimes does not have enough accuracy in its synthesis. In contrast, no remarkable differences are found within the range of 14 to 18 nucleotides. The chain length of the clamp primer of the present embodiment is an optimum and sufficient one.

### <Embodiment 11>

Embodiment 11 will be explained. Embodiment 11 mainly relates to claim 13. In summary, this embodiment is a method for detecting a mutated gene characterized in that it is based on any one of embodiments 1 to 10 and the mutated gene is a mutated gene of human EGFR.

In the present embodiment, explanations for the same requirements and the like as those of any one of embodiments 1 to 10 will not be repeated, but only requirements and the like characteristic of the present embodiment will be explained hereinafter.

### (Preparation of gene pool for detection)

As the gene pool used in the present embodiment, genomic DNA or cDNA obtained from a subject to examine the presence or absence of mutated genes of human EGFR may be used. The gene pool may be preferably obtained form any of the specimens generally used in the diagnosis of lung cancer, for example, sputum, pleural effusion, bronchial lavage fluid such as bronchoalveolar lavage fluid, part of tissues obtained by surgery, or pathologic tissue slices. A method for preparing genomic DNA or mRNA from these specimens may be commonly used ones such as an alkali method. Further, commercially available kits for preparing DNA or mRNA may be used. A method for preparing cDNA from mRNA may be a commonly used one using RT. A commercially available cDNA preparation kit may be used.

### (Regarding known mutated genes of EGFR)

As shown in non-patent literatures 4 and 5, the following 11 mutations have been reported as EFGR gene mutations derived from NSCLC patients until now. The 11 mutations are mainly divided into 4 point mutations and 7 deletion mutations. All of the 11 mutations are present in one of exon 18, exon 19, and exon 21 which encode the intracellular tyrosine kinase domain of EGFR. Another point mutation is known in exon 20, as shown in non-patent literature 6.

1. G719C: A point mutation in which the 2155th base G (using the nucleotide number on cDNA when A of the initiation codon is regarded as the first base. The same number system is used below.) which is encoded in exon 18 is substituted with T, and as a result, the 719th glycine residue of human EGFR protein is substituted with cystein residue.

2. G719S: A point mutation in which the 2155th base G encoded in exon 18 is substituted with A, and as a result, the 719th glycine residue of human EGFR protein is substituted with serine residue.

3. E746-A750del-1: A deletion mutation in which 15 bases from the 2235th base G to 2249th base C encoded in exon 19 are deleted, and as a result, the 746th glutamic acid residue to 750th alanine residue of human EGFR protein are deleted.

4. E746-A750del-2: A deletion mutation in which 15 bases from the 2236th base G to 2250th base A encoded in exon 19 are deleted, and as a result, the 746th glutamic acid residue to 750th alanine residue of human EGFR protein are deleted.

5. L747-A750delT751S: A deletion mutation in which 12 bases from the 2240th base T to 2251st base A encoded in exon 19 are deleted, and as a result, the 747th leucine residue to 750th alanine residue of human EGFR protein are deleted and the 751st thymidine residue is replaced with serine residue.

6. L747-S752delP753S: A deletion mutation in which 16 bases from the 2240th base T to 2257th base C encoded in exon 19 are deleted, and as a result, the 747th leucine residue to 752nd serine residue of human EGFR protein are deleted and the 753rd proline residue is replaced with serine residue.

7. L747-E740delA750P: A deletion mutation in which 9 bases from the 2239th base T to 2247th base A encoded in exon 19 are deleted, and as a result, the 747th leucine residue to 740th glutamic acid residue of human EGFR protein are deleted and the 750th alanine residue is replaced with proline residue.

8. L747-S752delE746V: A deletion mutation in which 18 bases from the 2238th base A to 2255th base C encoded in exon 19 are deleted, and as a result, the 747th leucine residue to 752nd serine residue of human EGFR protein are deleted and the 746th glutamic acid residue is replaced with valine residue.

9. S752-I759del: A deletion mutation in which 24 bases from the 2254th base T to 2277th base C encoded in exon 19 are deleted, and as a result, the 752nd serine residue to 759th isoleucine of human EGFR protein are deleted.

10. L858R: A point mutation in which the 2573rd base T encoded in exon 21 is substituted with G, and as a result, the 858th lysine residue of human EGFR protein is substituted with arginine residue.

11. L861Q: A point mutation in which the 2582nd base T encoded in exon 21 is substituted with A, and as a result, the 861st lysine residue of human EGFR protein is substituted with glutamine residue.

As described above, a normal PNA-LNA-PCR clamp method using a gene pool as a starting material (the sample to be assayed) is explained based on concrete embodiments 1 to 11. In the present invention, after a region comprising a target site of a mutated gene is selectively amplified using a gene pool as a starting material and in the presence of a specific clamp primer (pre-amplification step), the main amplification step of a normal PNA-LNA-PCR clamp method may be performed using the resulting amplified product or part thereof (preferably a dilution of the resulting amplified product) as a template. In this regard, various known highly sensitive detection methods may be used instead of the PNA-LNA-PCR clamp method in the present invention, as described above.

The "clamp primer" used in the pre-amplification step (hereinafter referred to as clamp primer for pre-amplification step) is not limited, so long as it is composed of PNA which hybridizes with all or part of a target site having a sequence of a wild-type gene or a sequence complementary to the wild-type gene. With respect to the clamp primer for pre-amplification step, explanations for the same requirements and the like as those of the clamp primer for main amplification step will not be repeated, but only requirements and the like characteristic of the clamp primer for pre-amplification step will be explained hereinafter.

The nucleotide sequence of the clamp primer for pre-amplification step may be the same as or different from that of the clamp primer for main amplification step.
For example, when the clamp primer for main amplification step has a sequence which hybridizes with all or part of a target site having a sequence (for example, a sense sequence) of a wild-type gene, the clamp primer for pre-amplification step may have a sequence which hybridizes with all or part of a target site having the sequence (sense sequence) of the wild-type gene, or a sequence which hybridizes with all or part of a target site having a sequence (antisense sequence) complementary to the wild-type gene. In the former case, the sequence of the clamp primer for main amplification step and the sequence of the clamp primer for pre-amplification step may be anexact match or a partial match (i.e., partially overlap), or may not have any overlapping sequences.
In contrast, when the clamp primer for main amplification step has a sequence which hybridizes with all or part of a target site having a sequence (antisense sequence) complementary to the wild-type gene, the clamp primer for pre-amplification step may have a sequence which hybridizes with all or part of a target site having a sequence (sense sequence) of the wild-type gene, or a sequence which hybridizes with all or part of a target site having a sequence (antisense sequence) complementary to the wild-type gene. In the latter case, the sequence of the clamp primer for main amplification step and the sequence of the clamp primer for pre-amplification step may be exact match or partial match, or may not have any overlapping sequences.
The concentration of the clamp primer used in the pre-amplification step varies in accordance with the amount of a gene pool used as the template and the amount of the wild-type gene contained in the gene pool, and is generally 0.01 µmol/L to 10 µmol/L, preferably 0.05 µmol/L to 5 µmol/L, and more preferably 0.1 µmol/L to 1 µmol/L.

With respect to the "amplification primer" and the "reaction solution for gene amplification" used in the pre-amplification step, explanations for the same requirements and the like as those used in the main amplification step will not be repeated, but only requirements and the like characteristic in the pre-amplification step will be explained hereinafter.

### (Pre-amplification step)

A gene pool containing the wild-type gene and the mutated gene, the PNA clamp primer, and the amplification primer are allowed to coexist in the reaction solution for gene amplification. Since wild-type genes are generally present in a gene pool in large amounts, the clamp primer is added in slight excess amount. As a result, the clamp primer, the mutation probe and the amplification primer hybridize with the respective corresponding complementary sequences on the genes to be detected. Since the target sites of the wild-type gene and the mutated gene have the same sequences except the mutated site, both the hybridizations to the target sites essentially compete with each other. However, since PNA exhibits a high specificity to its complementary nucleotide sequence, even if the mutation is a difference in only one nucleotide, almost all the clamp primer hybridizes with the target site on the wild-type gene.

Next, the resulting reaction solution for gene amplification is subjected to a primer elongation reaction using a gene amplification method. Since the clamp primer does not function as a primer, no elongation reaction occur. Therefore, the amplification of a detection region comprising the target site of the wild-type gene is suppressed by the hybridization of the clamp primer. In contrast, since the clamp primer does not hybridize with almost all the mutated gene, there is no factor which inhibits the primer elongation, and therefore, an elongation reaction by the amplification primer occurs, and a detection region comprising the target site of the mutated gene is selectively amplified.

All or part of the resulting amplified product (or an appropriate dilution of the resulting amplified product) is used as the template to perform the subsequent main amplification step. For example, the resulting amplified product is generally diluted 100-fold to 10,000-fold with a nuclease-free solvent which does not inhibit the amplification in the main amplification step, such as sterilized ultrapure water, and used as the template.
The pre-amplification step may be performed twice or more before the main amplification step.

### <Embodiment 12>

Embodiment 12 will be explained. This embodiment mainly relates to claim 12. The present embodiment is a method for selectively detecting a detection region comprising a target site of a mutated gene in step (2) (i.e., the main amplification step) of claim 1, and is commonly used detection technique in the present technical field. Examples of the detection technique include an invader method, a TaqMan-PCR method, a Scorpion ARMS method, a DNA chip method, a PCR-RFLP method, a PCR-SSCP method, a PCR-HRM method, a gFCS method, or a Southern blotting method. The invader method and the Scorpion ARMS method, which are known to have a high detection sensitivity, are preferable.

### EXAMPLES

The present invention now will be further illustrated by, but is by no means limited to, the following Examples.

### <<Example 1: Evaluation of effects of addition of PNA clamp primer in pre-amplification step of DNA template used in highly sensitive detection method (1)>>

In this Example, mutated EGFR genes contained in human genomic DNA pools, deletion mutation EGFR gene (E746-A750 deletion mutation in exon 19) and point mutation EGFR gene (L858R point mutation in exon 21), were used as a target to evaluate the effectiveness of the addition of a PNA clamp primer in a pre-amplification step of a DNA template used in a highly sensitive detection method (a PNA-LNA-PCR clamp method) and its detection sensitivity. The conditions of the PNA-LNA-PCR clamp method were selected in accordance with Japanese Patent No. 4,216,266.

### (Materials)

Cell line PC9 (EGFR Exon19 E746-A750 deletion mutation) and cell line 11-18 (EGFR Exon21 L858R point mutation) purchased from the Coriell Institute were cultured by a conventional method, and genomic DNAs extracted from the cells using a commercially available kit (DNeasy Blood & Tissue Kit (Qiagen)) were used as mutated EGFR genes. HapMap DNA purchased from the Coriell Institute was used as a wild-type EGFR gene.

These DNAs were used to prepare 17-step dilution series (15 µL each) as shown in Table 1 (PC9 dilution series, Exon19 E746-A750 deletion mutation) and Table 2 (11-18 dilution series, Exon21 L858R point mutation).
More particularly, the copy number of the total EGFR genes was regarded as 500 copies (corresponding to 1650 pg as the amount of human genomic DNA), and the dilution series were prepared so that the percentage of the copy number of each mutated EGFR gene to the copy number of the total EGFR genes became 50%, 10%, 2%, 0.4%, and 0%.
Similarly, the dilution series were prepared:
when the copy number of the total EGFR genes was regarded as 100 copies (corresponding to 330 pg as the amount of human genomic DNA), so that the percentage of the copy number of each mutated EGFR gene to the copy number of the total EGFR genes became 50%, 10%, 2%, 0.4%, and 0%;
when the copy number of the total EGFR genes was regarded as 20 copies (corresponding to 66 pg as the amount of human genomic DNA), so that the percentage of the copy number of each mutated EGFR gene to the copy number of the total EGFR genes became 50%, 10%, 2%, and 0%; and
when the copy number of the total EGFR genes was regarded as 5 copies (corresponding to 16.5 pg as the amount of human genomic DNA), so that the percentage of the copy number of each mutated EGFR gene to the copy number of the total EGFR genes became 50%, 10%, and 0%.

PC9 dilution series (Exon19 deletion)

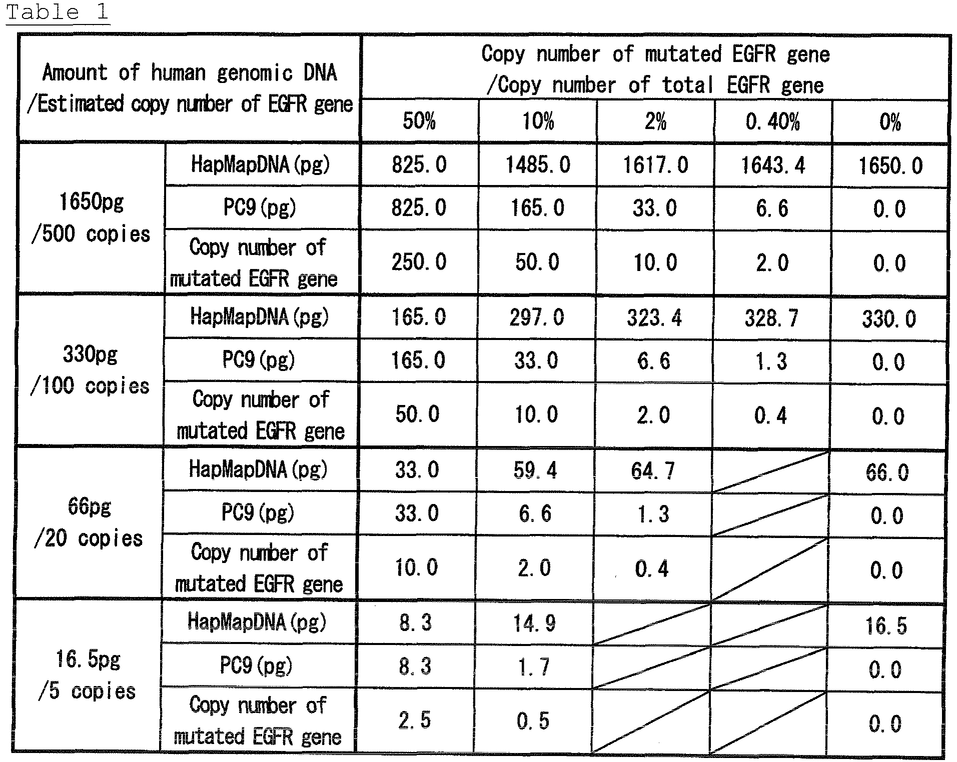

11-18 dilution series (Exon21-L858R)

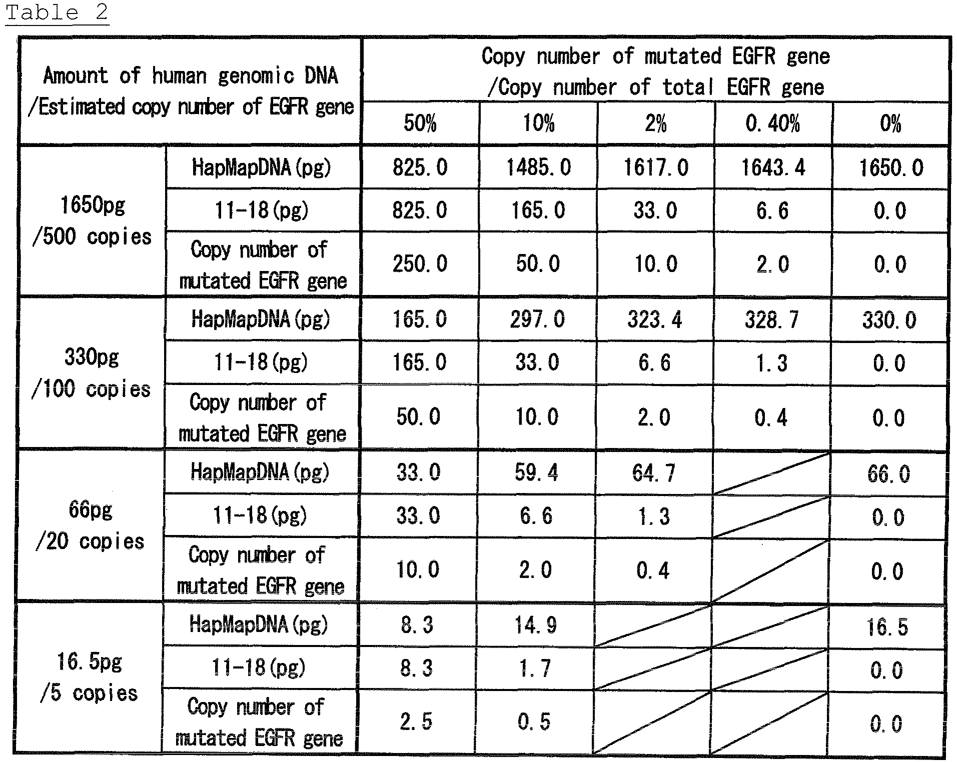

### (Performance of pre-amplification step of DNA template used in PNA-LNA-PCR clamp method)

The 17-step dilution series DNAs were added to reaction solutions for gene amplification to perform a pre-amplification step under the following conditions of series (1) to (4) described below. Series (1) and series (2) were ones for detecting the E746-A750 deletion mutation in exon 19 of the EGFR gene, and series (3) and series (4) were ones for detecting the L858R point mutation in exon 21 of the EGFR gene. Series (1) and series (3) were ones in which the PNA clamp primer was not added (PNA-), and series (2) and series (4) were ones in which the PNA clamp primer was added (PNA+) .

### (1) Detection of Exon19 E746-A750 deletion mutation in EGFR gene (PNA-)

A reaction solution for gene amplification of series (1) was prepared by mixing the following reagents:
10xPCR buffer (Qiagen: attached to HotStarTaq polymerase enzyme product): 2 µL,
10 mmol/L dNTPmix (= dATP, dTTP, dGTP, dCTP): 0.4 µL,
25 µmol/L amplification forward primer ex19-out-F (nucleotide sequence: 5'-GTGCATCGCTGGTAACATCC-3': SEQ ID NO: 1): 0.4 µL,
25 µmol/L amplification reverse primer ex19-out-B (nucleotide sequence: 5'-TGAGGTTCAGAGCCATGGAC-3': SEQ ID NO: 2): 0.4 µL, and
0.5 U HotStarTaq polymerase (Qiagen),
and adding sterile water to adjust the volume to 5 µL.

### (2) Detection of Exon19 E746-A750 deletion mutation in EGFR gene (PNA+)

A reaction solution for gene amplification of series (2) was prepared by mixing the following reagents:
10xPCR buffer (Qiagen: attached to HotStarTaq polymerase enzyme product): 2 µL,
10 mmol/L dNTPmix (= dATP, dTTP, dGTP, dCTP): 0.4 µL,
25 µmol/L amplification forward primer ex19-out-F (nucleotide sequence: 5'-GTGCATCGCTGGTAACATCC-3': SEQ ID NO: 1): 0.4 µL,
25 µmol/L amplification reverse primer ex19-out-B (nucleotide sequence: 5'-TGAGGTTCAGAGCCATGGAC-3': SEQ ID NO: 2): 0.4 µL,
100 µmol/L PNA clamp primer Delc1 (Panagene: nucleotide sequence: NH₂-AGATGTTGCTTCTCTTAA-CONH₂): 1 µL, and
0.5 U HotStarTaq polymerase (Qiagen),
and adding sterile water to adjust the volume to 5 µL.

### (3) Detection of Exon21 L858R point mutation in EGFR gene (PNA-)

A reaction solution for gene amplification of series (3) was prepared by mixing the following reagents:
10xPCR buffer (Qiagen: attached to HotStarTaq polymerase enzyme product): 2 µL,
10 mmol/L dNTPmix (= dATP, dTTP, dGTP, dCTP): 0.4 µL,
25 µmol/L amplification forward primer ex21-F (nucleotide sequence: 5'-GCATGAACTACTTGGAGGAC-3': SEQ ID NO: 3): 0.4 µL,
25 µmol/L amplification reverse primer ex21-B (nucleotide sequence: 5'-ACCTAAAGCCACCTCCTTAC-3': SEQ ID NO: 4): 0.4 µL, and
0.5 U HotStarTaq polymerase (Qiagen),
and adding sterile water to adjust the volume to 5 µL.

### (4) Detection of Exon21 L858R point mutation in EGFR gene (PNA+)

A reaction solution for gene amplification of series (4) was prepared by mixing the following reagents:
10xPCR buffer (Qiagen: attached to HotStarTaq polymerase enzyme product): 2 µL,
10 mmol/L dNTPmix (= dATP, dTTP, dGTP, dCTP): 0.4 µL,
25 µmol/L amplification forward primer ex21-F (nucleotide sequence: 5'-GCATGAACTACTTGGAGGAC-3': SEQ ID NO: 3): 0.4 µL,
25 µmol/L amplification reverse primer ex21-B (nucleotide sequence: 5'-ACCTAAAGCCACCTCCTTAC-3': SEQ ID NO: 4): 0.4 µL,
100 µmol/L PNA clamp primer L858c (Panagene: nucleotide sequence: NH₂-TTTGGCCAGCCCAA-CONH₂): 1 µL, and
0.5 U HotStarTaq polymerase (Qiagen),
and adding sterile water to adjust the volume to 5 µL.

The 17-step dilution series were separately added to the reaction solutions for gene amplification of series (1) to series (4), and DNAs were amplified using a thermal cycler (GeneAmp9700 (Applied BioSystems)). The DNA amplification was performed by heating at 95°C for 15 minutes, repeating a cycle composed of reactions at 94°C for 30 seconds, at 55°C for 30 seconds, and at 72°C for 30 seconds 40 times, and heating at 72°C for 10 minutes, and the amplified products were stored at 10°C.

### (Performance of PNA-LNA-PCR clamp method using pre-amplified DNA templates)

The amplified DNA solutions of series (1) to (4) were diluted 2000-fold with sterile water, and parts of the dilutions were used as templates in a PNA-LNA-PCR clamp method.

### (A) Detection of Exon19 E746-A750 deletion mutation in EGFR gene

The previously prepared and diluted DNA solutions of series (1) and (2) were analyzed by a PNA-LNA-PCR clamp method to detect the E746-A750 deletion mutation in exon 19 of the EGFR gene.

More particularly, a reaction solution for gene amplification of the PNA-LNA-PCR clamp method was prepared by mixing the following reagents:
Premix Ex Taq Hot Start Version (Takara-Bio): 12.5 µL,
100 µmol/L amplification forward primer ex19-out-F (nucleotide sequence: 5'-GTGCATCGCTGGTAACATCC-3'): 0.05 µL,
100 µmol/L amplification reverse primer ex19-out-B (nucleotide sequence: 5'-TGAGGTTCAGAGCCATGGAC-3'): 0.05 µL,
100 µmol/L PNA clamp primer Delc1 (Panagene: nucleotide sequence: NH₂-AGATGTTGCTTCTCTTAA-CONH₂): 1 µL,
100 µmol/L LNA-total probe ALL1F: 0.025 µL,
100 µmol/L LNA-mutation probe E746-A750del-1pF: 0.025 µL, and
2000-fold diluted DNA solution: 5 µL,
   and adding sterile water to adjust the volume to 25 µL.
LNA-total probe ALL1F was designed so that a sequence common to the wild-type EGFR and the mutated EGFR could be detected.

The synthesis of all the probes was outsourced to IDT. The details of the probes are shown below.
Sequence of LNA-total probe ALL1F:
5'-(Cy5)ttaaCtTTCtCaCct(BHQ2)-3'
Sequence of LNA-mutation probe E746-A750del-1pF:
5'-(6FAM)ctatcaaAaCatctccgaaagc(BHQ1)-3'
The small letters in the sequences represent DNA, the capital letters represent LNA, 6FAM and Cy5 are fluorescent dyes, and BHQ1 and BHQ2 are quenchers.

The PNA-LNA-PCR clamp method for detecting the E746-A750 deletion mutation in exon 19 of the EGFR gene was performed using a thermal cycler (Smart Cycler II (Cepheid Sunnyvale)). The PCR clamp reaction was performed by heating at 95°C for 10 seconds and repeating a cycle composed of reactions at 95°C for 3 seconds and at 62°C for 30 seconds 60 times. The amplification was confirmed using an analysis software attached to the thermal cycler, and the positive standard of the LNA-total probe ALL1F was 5<Ct value<25, and the positive standard of the LNA-mutation probe E746-A750de1-1pF was 5<Ct value<25. The LNA-total probe ALL1F was used to confirm the amplification of the total EGFR genes, and to confirm that the PNA-LNA-PCR clamp method had been normally performed. The LNA-mutation probe E746-A750del-1pF was used to examine whether or not the gene mutation of the EGFR could be detected.

### (Results in detection of Exon19 E746-A750 deletion mutation in EGFR gene)

The 17-step diluted DNAs were used as described above to compare the effects of the addition of the PNA clamp primer in the pre-amplification. In this evaluation, the procedure was independently repeated three times, and cases in which the mutation were detected twice or more was judged as "mutation was detected". The results are shown in Table 3. An increase in detection sensitivity by the addition of the PNA clamp primer in the pre-amplification was confirmed in the case in which 8.3 pg of HapMap DNA (containing the wild-type EGFR gene) and 8.3 pg of PC9-derived DNA (containing the mutated EGFR gene) were mixed in equal proportions (2.5 copies of the EGFR gene mutation in DNA corresponding to 5 cells as an estimated cell number).
In Table 3 (and Table 4 below), "o" indicates cases in which mutated EGFR was detected twice or more in the three experiments, and "×" indicates the other cases. The box shaded in gray indicates cases in which the effects of the PNA addition in the pre-amplification were confirmed.

### Exon19 E746-A750 deletion mutation

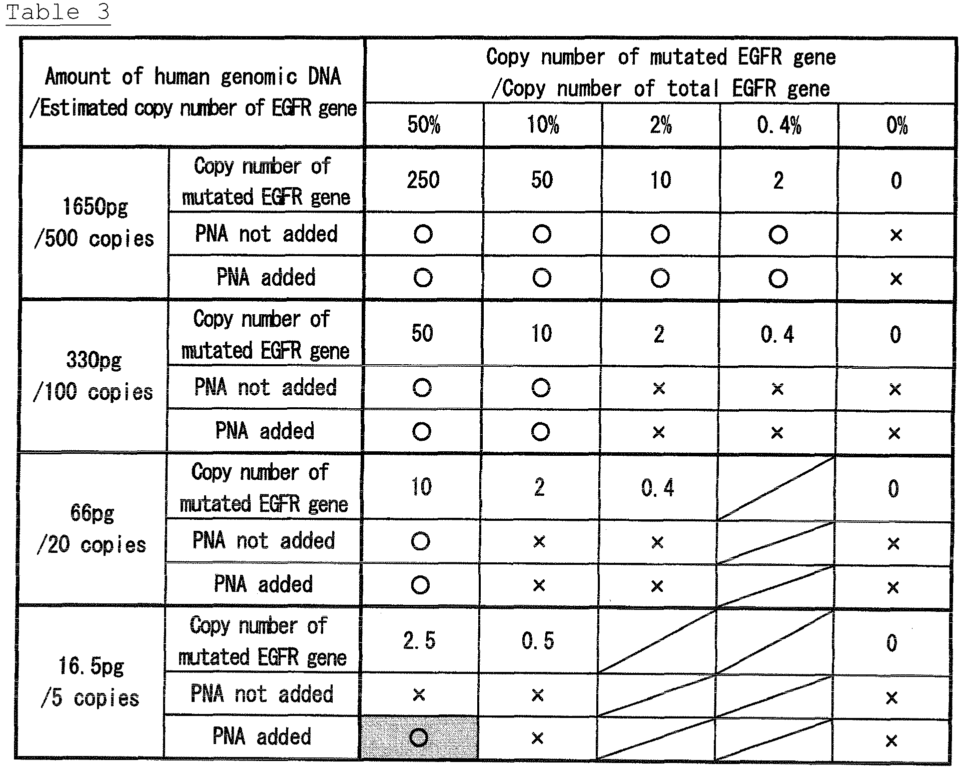

### (B) Detection of Exon21 L858R point mutation in EGFR gene

The previously prepared and diluted DNA solutions of series (3) and (4) were analyzed by a PNA-LNA-PCR clamp method to detect the L858R point mutation in exon 21 of the EGFR gene.

More particularly, a reaction solution for gene amplification of the PNA-LNA-PCR clamp method was prepared by mixing the following reagents:
Premix Ex Taq Hot Start Version (Takara-Bio): 12.5 µL,
100 µmol/L amplification forward primer ex21-F (nucleotide sequence: 5'-GCATGAACTACTTGGAGGAC-3'): 0.05 µL,
100 µmol/L amplification reverse primer ex21-B (nucleotide sequence: 5'-ACCTAAAGCCACCTCCTTAC-3'): 0.05 µL,
100 µmol/L PNA clamp primer L858c (Panagene: nucleotide sequence: NH₂-TTTGGCCAGCCCAA-CONH₂) : 1 µL,
100 µmol/L LNA-total probe ALL2F: 0.025 µL,
100 µmol/L LNA-mutation probe L858RpF: 0.025 µL, and
2000-fold diluted DNA solution: 5 µL,
   and adding sterile water to adjust the volume to 25 µL.
LNA-total probe ALL2F was designed so that a sequence common to the wild-type EGFR and the mutated EGFR could be detected.

The synthesis of all the probes was outsourced to IDT. The details of the probes are shown below.
Sequence of LNA-total probe ALL2F:
5'-(Cy5) ccaGgaaCgtaCtg (BHQ2)-3'
Sequence of LNA-mutation probe L858RpF:
5'-(6FAM) tttggccCgcccaa(BHQ1)-3'
The small letters in the sequences represent DNA, the capital letters represent LNA, 6FAM and Cy5 are fluorescent dyes, and BHQ1 and BHQ2 are quenchers.

The PNA-LNA-PCR clamp method for detecting the L858R point mutation in exon 21 of the EGFR gene was performed using a thermal cycler (Smart Cycler II (Cepheid Sunnyvale)). The PCR clamp reaction was performed by heating at 95°C for 10 seconds and repeating a cycle composed of reactions at 95°C for 3 seconds and at 62°C for 30 seconds 60 times. The amplification was confirmed using an analysis software attached to the thermal cycler, and the positive standard of the LNA-total probe ALL1F was 5<Ct value<25, and the positive standard of the LNA-mutation probe L858RpF was 5<Ct value<30. The LNA-total probe ALL2F was used to confirm the amplification of the total EGFR genes, and to confirm that the PNA-LNA-PCR clamp method had been normally performed. The LNA-mutation probe L858RpF was used to examine whether or not the gene mutation of the EGFR could be detected.

### (Results in detection of Exon21 L858R point mutation in EGFR gene)

The 17-step diluted DNAs were used as described above to compare the effects of the addition of the PNA clamp primer in the pre-amplification. In this evaluation, the procedure was independently repeated three times, and cases in which the mutation was detected twice or more were judged as "mutation was detected". The results are shown in Table 4. An increase in detection sensitivity by the addition of the PNA clamp primer in the pre-amplification was confirmed in the case in which 323.4 pg of HapMap DNA (containing the wild-type EGFR gene) and 6.6 pg of 11-18-derived DNA (containing the mutated EGFR gene) were mixed (2 copies of the EGFR gene mutation in DNA corresponding to 100 cells as an estimated cell number).

### Exon21 L858R point mutation

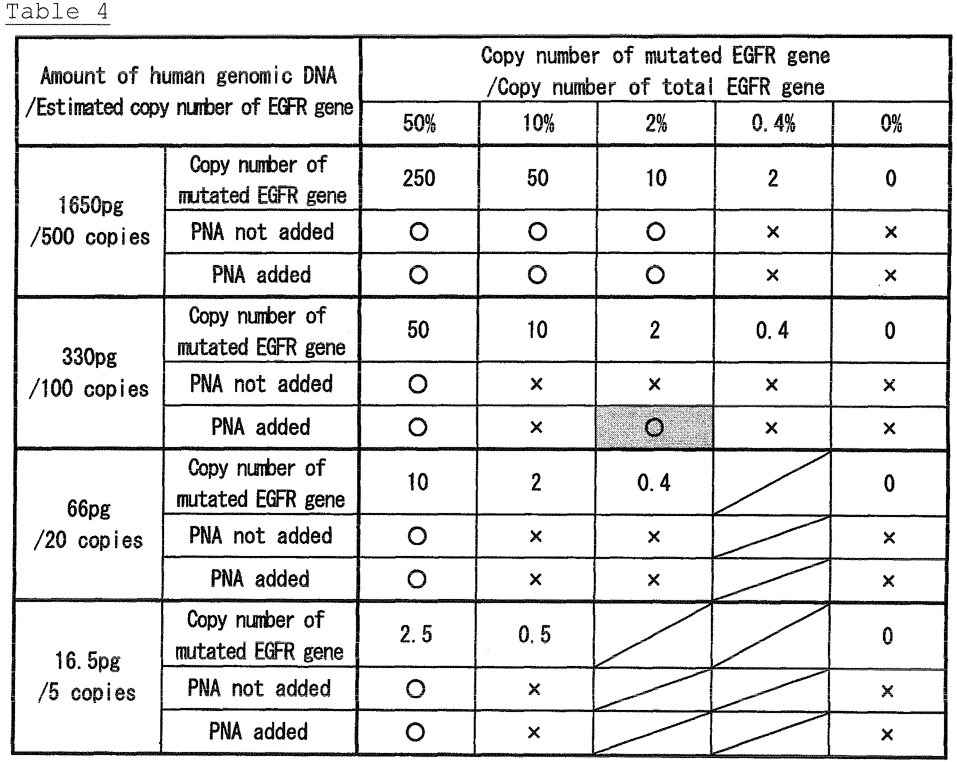

### <<Example 2: Evaluation of effects of addition of PNA clamp primer in pre-amplification step of DNA template used in highly sensitive detection method (2)>>

In this Example, a mutated EGFR gene contained in human genomic DNA pools, deletion mutation EGFR gene (E746-A750 deletion mutation in exon 19), was used as a target to evaluate the effectiveness of the addition of a PNA clamp primer in a pre-amplification step of a DNA template used in a highly sensitive detection method (a PNA-LNA-PCR clamp method) and its detection sensitivity. The conditions of the PNA-LNA-PCR clamp method were selected in accordance with Japanese Patent No. 4, 216, 266.

### (Materials)

In a similar fashion to that described in Example 1, cell line PC14 (EGFR Exon19 E746-A750 deletion mutation) purchased from the Coriell Institute was cultured by a conventional method, and genomic DNA extracted from the cells using a commercially available kit (DNeasy Blood & Tissue Kit (Qiagen)) was used as mutated EGFR genes. HapMap DNA purchased from the Coriell Institute was used as a wild-type EGFR gene.

These DNAs were used to prepare 6-step dilution series (15 µL each) as shown in Table 5 (PC14 dilution series, Exon19 E746-A750 deletion mutation).
More particularly, the copy number of the total EGFR genes was regarded as 10000 copies (corresponding to 33000 pg as the amount of human genomic DNA), and the dilution series were prepared so that the percentage of the copy number of the mutated EGFR gene to the copy number of the total EGFR genes became 2%, 1%, 0. 2%, 0. 1%, 0. 02%, and 0%.

### PC14 dilution series (Exon19 deletion)

**Table 5**

| Amount of human genomic DNA /Estimated copy number of EGFR gene | Copy number of mutated EGFR gene /Copy number of total EGFR gene | | | | | |
|---|---|---|---|---|---|---|
| | 2% | 1% | 0.2% | 0.1% | 0.02% | 0% |
| HapMapDNA (pg) | 32340 | 32670 | 32934 | 32967 | 32993.4 | 33000 |
| PC14 (pg) | 660 | 330 | 66 | 33 | 6.6 | 0 |
| Copy number of mutated EGFR gene | 200 | 100 | 20 | 10 | 2 | 0 |

### (Performance of pre-amplification step of DNA template used in PNA-LNA-PCR clamp method)

The 6-step dilution series DNAs were added to reaction solutions for gene amplification of series (1) and (2) to perform a pre-amplification step. Series (1) was one in which the PNA clamp primer was not added (PNA-), and series (2) was one in which the PNA clamp primer was added (PNA+).

### Preparation of reaction solution for gene amplification of series (1) (PNA-)

A reaction solution for gene amplification of series (1) was prepared by mixing the following reagents:
10xPCR buffer (Qiagen: attached to HotStarTaq polymerase enzyme product): 2 µL,
10 mmol/L dNTPmix (= dATP, dTTP, dGTP, dCTP): 0.4 µL,
25 µmol/L amplification forward primer ex19-out-F (nucleotide sequence: 5'-GTGCATCGCTGGTAACATCC-3': SEQ ID NO: 1): 0.4 µL,
25 µmol/L amplification reverse primer ex19-out-B (nucleotide sequence: 5'-TGAGGTTCAGAGCCATGGAC-3': SEQ ID NO: 2): 0.4 µL, and
0.5 U HotStarTaq polymerase (Qiagen),
   and adding sterile water to adjust the volume to 19 µL.

### Preparation of reaction solution for gene amplification of series (2) (PNA)+)

A reaction solution for gene amplification of series (2) was prepared in a similar fashion to that described in series (1), except that
100 µmol/L PNA clamp primer Delc1 (Panagene: nucleotide sequence: NH₂-AGATGTTGCTTCTCTTAA-CONH₂): 1 µL was added.

The 6-step dilution series DNAs (1 µL) were separately added to the previously-prepared reaction solutions for gene amplification of series (1) and (2), and DNAs were amplified using a thermal cycler (GeneAmp9700 (Applied BioSystems)). The DNA amplification was performed by heating at 95°C for 15 minutes, repeating a cycle composed of reactions at 94°C for 30 seconds, at 55°C for 30 seconds, and at 72°C for 30 seconds 40 times, and heating at 72°C for 10 minutes, and the amplified products were stored at 10°C.

The amplified DNA solutions of series (1) and (2) were diluted 5000-fold with sterile water, and parts of the dilutions were used as templates in a PNA-LNA-PCR clamp method.

### (Performance of PNA-LNA-PCR clamp method using pre-amplified DNA templates)

The previously prepared and diluted DNA solutions of series (1) and (2) were analyzed by a PNA-LNA-PCR clamp method to detect the E746-A750 deletion mutation in exon 19 of the EGFR gene.

More particularly, a reaction solution for gene amplification of the PNA-LNA-PCR clamp method was prepared by mixing the following reagents:
Premix Ex Taq Hot Start Version (Takara-Bio): 12.5 µL,
100 µmol/L amplification forward primer ex19-in-F (nucleotide sequence: 5'-TGTCATAGGGACTCTGGATCC-3': SEQ ID NO: 5): 0.05 µL,
100 µmol/L amplification reverse primer ex19-in-R (nucleotide sequence: 5'-AGCAGAAACTCACATCGAG-3': SEQ ID NO: 6): 0.05 µL,
100 µmol/L PNA clamp primer Delc1: 1 µL,
100 µmol/L LNA-total probe ALL1F: 0.025 µL,
100 µmol/L LNA-mutation probe E746-A750del-1pF: 0.025 µL, and
5000-fold diluted DNA solution: 5 µL,
   and adding sterile water to adjust the volume to 25 µL.
LNA-total probe ALL1F was designed so that a sequence common to the wild-type EGFR and the mutated EGFR could be detected.

The synthesis of all the probes was outsourced to IDT. The details of the probes are shown below.
Sequence of LNA-total probe ALL1F:
5'-(Cy5)ttaaCtTTCtCaCct(BHQ2)-3'
LNA-mutation probe E746-A750del-1pF:
5'-(6FAM)ctatcaaAaCatctccgaaagc(BHQ1)-3'
The small letters in the sequences represent DNA, the capital letters represent LNA, 6FAM and Cy5 are fluorescent dyes, and BHQ1 and BHQ2 are quenchers.

The PNA-LNA-PCR clamp method for detecting the E746-A750 deletion mutation in exon 19 of the EGFR gene was performed using a thermal cycler (Smart Cycler II (Cepheid Sunnyvale)). The PCR clamp reaction was performed by heating at 95°C for 10 seconds and repeating a cycle composed of reactions at 95°C for 3 seconds and at 62°C for 30 seconds 45 times. The amplification was confirmed using an analysis software attached to the thermal cycler, and the positive standard of the LNA-total probe ALL1F was 5<Ct value<25, and the positive standard of the LNA-mutation probe E746-A750del-1pF was 5<Ct value<25. The LNA-total probe ALL1F was used to confirm the amplification of the total EGFR genes, and to confirm that the PNA-LNA-PCR clamp method had been normally performed. The LNA-mutation probe E746-A750del-1pF was used to examine whether or not the gene mutation of the EGFR could be detected.

### (Results in detection of Exon19 E746-A750 deletion mutation in EGFR gene)

The 6-step diluted DNAs were used as described above to compare the effects of the addition of the PNA clamp primer in the pre-amplification. In this evaluation, cases in which the mutation was detected were judged as "mutation was detected". The results are shown in Table 6. An increase in detection sensitivity by the addition of the PNA clamp primer in the pre-amplification was confirmed in the case in which 32967 pg of HapMap DNA (containing the wild-type EGFR gene) and 33 pg of PC14-derived DNA (containing the mutated EGFR gene) were mixed (2 copies of the EGFR gene mutation in DNA corresponding to 5000 cells as an estimated cell number). In Table 6, "o" indicates cases in which the mutated EGFR was detected, and "x" indicates cases in which the mutated EGFR was not detected.
Further, mutations such as L858R were evaluated to obtain the same results.

### Exon19 E746-A750 deletion mutation

**Table 6**

| Amount of human genomic DNA /Estimated copy number of EGFR gene | Copy number of mutated EGFR gene /Copy number of total EGFR gene | | | | | |
|---|---|---|---|---|---|---|
| | 2% | 1% | 0.2% | 0.1% | 0.02% | 0% |
| HapMapDNA (pg) | 32340 | 32670 | 32934 | 32967 | 32993.4 | 33000 |
| PC14 (pg) | 660 | 330 | 66 | 33 | 6.6 | 0 |
| Copy number of mutated EGFR gene | 200 | 100 | 20 | 10 | 2 | 0 |
| PNA- | ○ | ○ | × | × | × | × |
| PNA+ | ○ | ○ | ○ | ○ | × | × |

### <<Example 3: Examination of preferable conditions in pre-amplification step (1)>>

In this example, a mutated EGFR gene contained in human genomic DNA pools, deletion mutation EGFR gene (E746-A750 deletion mutation in exon 19), was used as a target to evaluate a preferable concentration of a PNA clamp primer in a pre-amplification step of a DNA template used in a highly sensitive detection method (a PNA-LNA-PCR clamp method).

### (Materials)

In a similar fashion to that described in Example 1, cell line PC14 (EGFR Exon19 E746-A750 deletion mutation) purchased from the Coriell Institute was cultured by a conventional method, and genomic DNA extracted from the cells using a commercially available kit (DNeasy Blood & Tissue Kit (Qiagen)) was used as the mutated EGFR gene. HapMap DNA purchased from the Coriell Institute was used as the wild-type EGFR gene.

These DNAs were used to prepare 5-step dilution series (15 µL each) as shown in Table 7 (PC14 dilution series, Exon19 E746-A750 deletion mutation).
More particularly, the copy number of the total EGFR genes was regarded as 100 copies (corresponding to 330 pg as the amount of human genomic DNA), and the dilution series were prepared so that the percentage of the copy number of the mutated EGFR gene to the copy number of the total EGFR genes became 10%, 5%, 2%, 1%, and 0%.

### PC14 dilution series (Exon19 deletion)

**Table 7**

| Amount of human genomic DNA /Estimated copy number of EGFR gene | Copy number of mutated EGFR gene /Copy number of total EGFR gene | | | | |
|---|---|---|---|---|---|
| | 10% | 5% | 2% | 1% | 0% |
| HapMapDMA(pg) | 297 | 313.5 | 323.4 | 326.7 | 330 |
| PC14 (pg) | 33 | 16.5 | 6.6 | 3.3 | 0 |
| Copy number of mutated EGFR gene | 10 | 5 | 2 | 1 | 0 |

### (Performance of pre-amplification step of DNA template used in PNA-LNA-PCR clamp method)

The 5-step dilution series DNAs were added to reaction solutions for gene amplification of series (1) to (6) to perform a pre-amplification step. Series (1) was one in which the PNA clamp primer was not added (0 µmol/L PNA), series (2) was one in which 0.05 µmol/L of the PNA clamp primer was added (0.05 µmol/L PNA), series (3) was one in which 0.1 µmol/L of the PNA clamp primer was added (0.1 µmol/L PNA), series (4) was one in which 0.5 µmol/L of the PNA clamp primer was added (0.5 µmol/L PNA), series (5) was one in which 1 µmol/L of the PNA clamp primer was added (1 µmol/L PNA), and series (6) was one in which 5 µmol/L of the PNA clamp primer was added (5 µmol/L PNA).

### Preparation of reaction solution for gene amplification of series (1) (0 µmol/L PNA)

A reaction solution for gene amplification of series (1) was prepared in a similar fashion to that described in series (1) of Example 2.

### Preparation of reaction solution for gene amplification of series (2) (0.05 µmol/L PNA)

A reaction solution for gene amplification of series (2) was prepared in a similar fashion to that described in series (1), except that
1 µmol/L PNA clamp primer Delc1: 1 µL
was added.

### Preparation of reaction solution for gene amplification of series (3) (0.1 µmol/L PNA)

A reaction solution for gene amplification of series (3) was prepared in a similar fashion to that described in series (1), except that
2 µmol/L PNA clamp primer Delc1: 1 µL
was added.

### Preparation of reaction solution for gene amplification of series (4) (0.5 µmol/L PNA)

A reaction solution for gene amplification of series (2) was prepared in a similar fashion to that described in series (4), except that
10 µmol/L PNA clamp primer Delc1: 1 µL
was added.

### Preparation of reaction solution for gene amplification of series (5) (1 µmol/L PNA)

A reaction solution for gene amplification of series (2) was prepared in a similar fashion to that described in series (5), except that
20 µmol/L PNA clamp primer Delc1: 1 µL
was added.

### Preparation of reaction solution for gene amplification of series (6) (5 µmol/L PNA)

A reaction solution for gene amplification of series (2) was prepared in a similar fashion to that described in series (6), except that
100 µmol/L PNA clamp primer Delc1: 1 µL
was added.

The 6-step dilution series DNAs (1 µL) were separately added to the previously-prepared reaction solutions for gene amplification of series (1) to (6), and DNAs were amplified under the similar conditions to that described in Example 2.

The amplified DNA solutions of series (1) to (6) were diluted 5000-fold with sterile water, and parts of the dilutions were used as templates in a PNA-LNA-PCR clamp method.

### (Performance of PNA-LNA-PCR clamp method using pre-amplified DNA templates)

The previously prepared and diluted DNA solutions of series (1) to (6) were analyzed by a PNA-LNA-PCR clamp method for detecting the E746-A750 deletion mutation in exon 19 of the EGFR gene, in a similar fashion to that described in Example 2.

### (Results in detection of Exon19 E746-A750 deletion mutation in EGFR gene)

The 5-step diluted DNAs were used as described above to compare the effects of the addition of the PNA clamp primer in the pre-amplification. In this evaluation, cases in which the mutation was detected were judged as "mutation was detected". The results are shown in Table 8. An increase in detection sensitivity by the addition of the PNA clamp primer (0.05 to 5 µmol/L PNA) in the pre-amplification was confirmed in the case in which 326.7 pg of HapMap DNA (containing the wild-type EGFR gene) and 3.3 pg of PC14-derived DNA (containing the mutated EGFR gene) were mixed (1 copy of the EGFR gene mutation in DNA corresponding to 50 cells as an estimated cell number), or the case in which 323.4 pg of HapMap DNA (containing the wild-type EGFR gene) and 6.6 pg of PC14-derived DNA (containing the mutated EGFR gene) were mixed (2 copies of the EGFR gene mutation in DNA corresponding to 50 cells as an estimated cell number). In Table 8, "o" indicates cases in which the mutated EGFR was detected, and "×" indicates cases in which the mutated EGFR was not detected.

### Exon19 E746-A750 deletion mutation

**Table 8**

| Amount of human genomic DNA /Estimated copy number of EGFR gene | Copy number of mutated EGFR gene /Copy number of total EGFR gene | | | | |
|---|---|---|---|---|---|
| | 10% | 5% | 2% | 1% | 0% |
| HapMapDNA(pg) | 297 | 313.5 | 323.4 | 326.7 | 330 |
| PC14(pg) | 33 | 16.5 | 6.6 | 3.3 | 0 |
| Copy number of mutated EGFR gene | 10 | 5 | 2 | 1 | 0 |
| 0 µmol/L PNA | ○ | ○ | × | × | × |
| 0.05 *µ*mol/L PNA | ○ | ○ | × | ○ | × |
| 0.1 *µ*mol/L PNA | ○ | ○ | ○ | × | × |
| 0.5 *µ*mol/L PNA | ○ | ○ | ○ | ○ | × |
| 1 *µ*mol/L PNA | ○ | ○ | ○ | × | × |
| 5 *µ*mol/L PNA | ○ | ○ | ○ | × | × |

### <<Example 4: Examination of preferable conditions in pre-amplification step (2)>>

In this example, a mutated EGFR gene contained in human genomic DNA pools, deletion mutation EGFR gene (E746-A750 deletion mutation in exon 19), was used as a target to evaluate the presence or absence of the PNA clamp primer to be added and a preferable temperature for annealing in a pre-amplification step of a DNA template used in a highly sensitive detection method (a PNA-LNA-PCR clamp method).

### (Materials)

In a similar fashion to that described in Example 1, cell line PC14 (EGFR Exon19 E746-A750 deletion mutation) purchased from the Coriell Institute was cultured by a conventional method, and genomic DNA extracted from the cells using a commercially available kit (DNeasy Blood & Tissue Kit (Qiagen)) was used as the mutated EGFR gene. HapMap DNA purchased from the Coriell Institute was used as the wild-type EGFR gene.

In a similar fashion to that described in Example 3, these DNAs were used to prepare 5-step dilution series (15 µL each) as shown in Table 7 (PC14 dilution series, Exon19 E746-A750 deletion mutation).

### (Performance of pre-amplification step of DNA template used in PNA-LNA-PCR clamp method)

The 5-step dilution series DNAs were added to each reaction solution for gene amplification to perform a pre-amplification step under the conditions of series (1) to (8). Series (1) was one in which the PNA clamp primer was not added and the annealing temperature was 63°C (0 µmol/L PNA 63°C), series (2) was one in which the PNA clamp primer was added and the annealing temperature was 63°C (5 µmol/L PNA 63°C), series (3) was one in which the PNA clamp primer was not added and the annealing temperature was 60°C (0 µmol/L PNA 60°C), series (4) was one in which the PNA clamp primer was added and the annealing temperature was 60°C (5 µmol/L PNA 60°C), series (5) was one in which the PNA clamp primer was not added and the annealing temperature was 55°C (0 µmol/L PNA 55°C), series (6) was one in which the PNA clamp primer was added and the annealing temperature was 55°C (5 µmol/L PNA 55°C), series (7) was one in which the PNA clamp primer was not added and the annealing temperature was 50°C (0 µmol/L PNA 50°C), and series (8) was one in which the PNA clamp primer was added and the annealing temperature was 50°C (5 µmol/L PNA 50°C).

### Preparation of reaction solution for gene amplification of series (1), (3), (5), and (7) (0 µmol/L PNA)

A reaction solution for gene amplification of series (1), (3), (5), and (7) was prepared in a similar fashion to that described in series (1) of Example 2.

### Preparation of reaction solution for gene amplification of series (2), (4), (6), and (8) (5 µmol/L PNA)

A reaction solution for gene amplification of series (2), (4), (6), and (8) was prepared in a similar fashion to that described in series (1), except that
100 µmol/L PNA clamp primer Delcl: 1 µL
was added.

### Amplification of DNA

DNA was amplified under the same conditions as described in Example 2, except that 1 µL of the 5-step dilution series DNAs were separately added, and the annealing was performed at 50°C, 55°C, 60°C, and 63°C.

Parts of the amplified DNA solutions of series (1) to (8) were used as samples for automated electrophoresis.

### (Performance of automated electrophoresis using pre-amplified DNA templates)

The previously prepared DNA solutions of series (1) to (8) were analyzed by automated electrophoresis to detect the E746-A750 deletion mutation in exon 19 of the EGFR gene. QIAxcel (Qiagen) was used as an equipment for automated electrophoresis. The wild-type and deletion-type PCR amplification products can be distinguished from each other using this equipment.

More particularly, aliquots (6 µL) of the amplified DNA solutions of series (1) to (8) were put into a 96-well PCR plate (BMbio), and electrophoresis was performed using a QIAxel DNA Screening Kit (Qiagen) under the conditions of 5kV and 240 sec.

### (Results in detection of Exon19 E746-A750 deletion mutation in EGFR gene)

The 5-step diluted DNAs were used as described above to compare the effects of the addition of the PNA clamp primer in the pre-amplification. In this evaluation, cases in which the mutation was detected were judged as "mutation was detected". The results are shown in Table 9. Under the conditions at an annealing temperature of 50 to 63°C, an increase in detection sensitivity by the addition of the PNA clamp primer (0.05 to 5 µmol/L PNA) in the pre-amplification was confirmed in the case in which 326.7 pg of HapMap DNA (containing the wild-type EGFR gene) and 3.3 pg of PC14-derived DNA (containing the mutated EGFR gene) were mixed (1 copy of the EGFR gene mutation in DNA corresponding to 50 cells as an estimated cell number), or the case in which 323.4 pg of HapMap DNA (containing the wild-type EGFR gene) and 6.6 pg of PC14-derived DNA (containing the mutated EGFR gene) were mixed (2 copies of the EGFR gene mutation in DNA corresponding to 50 cells as an estimated cell number). In Table 9, "M" indicates the case in which the mutated EGFR was detected, "W" indicates cases in which the wild-type EGFR was detected, and "×" indicates cases in which neither was detected.

### Exon19 E746-A750 deletion mutation

**Table 9**

| Amount of human genomic DNA /Estimated copy number of EGFR gene | Copy number of mutated EGFR gene /Copy number of total EGFR gene | | | | |
|---|---|---|---|---|---|
| | 10% | 5% | 2% | 1% | 0% |
| HapMapDNA(pg) | 297 | 313.5 | 323.4 | 326.7 | 330 |
| PC14 (pg) | 33 | 16.5 | 6.6 | 3.3 | 0 |
| Copy number of mutated EGFR gene | 10 | 5 | 2 | 1 | 0 |
| 0 *µ*mol/L PNA 63°C | W | W | W | W | W |
| 5 *µ*mol/L PNA 63°c | M | M | M/W | × | × |
| 0 *µ*mol/L PNA 60°C | W | W | W | W | W |
| 5 *µ*mol/L PNA 60°C | M | M | × | × | W |
| 0 *µ*mol/L PNA 55°C | W | W | W | W | W |
| 5 *µ*mol/L PNA 55°C | M | M | M | × | × |
| 0 *µ*mol/L PNA 50°C | W | W | W | W | W |
| 5 *µ*mol/L PNA 50°C | M | M | × | M | × |

It was proved by the above-mentioned results that the detection sensitivity can further be increased by performing a pre-amplification step of a DNA template to be subjected to a PNA-LNA-PCR clamp method, in a reaction solution for gene amplification containing a PNA clamp primer. Therefore, in a clinical examination, this method enables the detection with higher sensitivity of DNA and RNA derived from, for example, a specimen into which many normal cells are incorporated such as pleural effusion with blood, tissues or sputum containing tumor cells at a low content, or tumor tissues which may be circulated in blood. Furthermore, since it is conjectured that these effects are obtained by increasing the content of the mutated DNA in the template to be subjected to the method for the detection of the mutated DNA by the pre-amplification with the PNA clamp primer, it is considered that this method can also be applied to the preparation of a DNA template to be subjected to highly-sensitive detection methods other than the PNA-LNA-PCR clamp method, such as an invader method or a Scorpion ARMS method.

### INDUSTRIAL APPLICABILITY

The present invention has very high clinical applicability as a genetic testing method for detecting a mutated gene. Furthermore, a dedicated device or the like are not required in performing the present invention, and devices and equipments that are installed in a general clinical testing room can be utilized. In conventional clinical testing by the diagnosis of pathologic tissue slices or cells, specialistic knowledge and skilled experience were required for judging the results, and thus the fostering of cytotechnologists and the like were essential for commercialization. However, according to the present invention, if only a certain technique for operation is acquired, particular specialistic knowledge and skilled experience are not required. Furthermore, the present invention makes the testing routine extremely easy, which enables a treatment of a large amount of samples within a short period. Therefore, commercialization is also easy.

In particular, the sensitivities of Gefitinib and Erlotinib can be directly searched by detecting a mutated gene of EGFR, which enables an early decision to administer Gefitinib or Erlotinib to an NSCLC patient, or avoidance of the administration of Gefitinib or Erlotinib to a patient having low sensitivity against Gefitinib or Erlotinib. Hence, the present invention enables a tailor-made therapy against NSCLC with the highest probability of success. Therefore, social need therefor is very high.
Although the present invention has been described with reference to specific embodiments, various changes and modifications obvious to those skilled in the art are possible without departing from the scope of the appended claims.

### FREE TEXT IN SEQUENCE LISTING

The nucleotide sequences of SEQ ID NOS: 1 to 6 of the sequence listing are primer sequences.

## Claims

1. A method for detecting the presence or absence of a known mutated gene contained in a gene pool, said method comprising the steps of:
(1) allowing
(1a) a clamp primer consisting of PNA which hybridizes with all or part of a target site having a sequence of a wild-type gene or a sequence complementary to the wild-type gene,
(1b) a primer capable of amplifying a region comprising a target site having a sequence of the mutated gene, and
(1c) the gene pool
to coexist in a reaction solution for gene amplification, and selectively amplifying the region comprising a target site of the mutated gene by a gene amplification method, and (2) selectively detecting a detection region comprising the target site of the mutated gene by a gene detection method, using an amplified product obtained in step (1) or part thereof as a template, to detect the presence or absence of the mutated gene.

2. The method according to claim 1, wherein step (2) is a step of:
allowing
(2a) a clamp primer consisting of PNA which hybridizes with all or part of a target site having a sequence of the wild-type gene, said clamp primer having a nucleotide sequence the same as or different from that of clamp primer (1a) used in step (1),
(2b) a mutation probe which hybridizes with all or part of a target site having a sequence of the mutated gene, and at least part of which consists of LNA, and
(2c) an amplified product obtained in step (1) or part thereof
to coexist in a reaction solution for gene amplification, and selectively amplifying a detection region comprising the target site of the mutated gene by a gene amplification method, to detect the presence or absence of the mutated gene.

3. The method according to claim 1, wherein step (2) is a step of:
allowing
(2a') a clamp primer consisting of PNA which hybridizes with all or part of a target site having a sequence complementary to the wild-type gene, said clamp primer having a nucleotide sequence the same as or different from that of clamp primer (1a) used in step (1),
(2b) a mutation probe which hybridizes with all or part of a target site having a sequence complementary to the mutated gene, and at least part of which consists of LNA, and
(2c) an amplified product obtained in step (1) or part thereof
to coexist in a reaction solution for gene amplification, and selectively amplifying a detection region comprising the target site of the mutated gene by a gene amplification method, to detect the presence or absence of the mutated gene.

4. The method according to claim 2 or 3, wherein the gene amplification method in step (2c) is a PCR method.

5. The method according to any one of claims 2 to 4, wherein the mutation probe comprises RNA.

6. The method according to any one of claims 2 to 5, wherein the mutation probe is labeled with a fluorescent substance at one terminus, and is labeled with a quencher which suppresses the fluorescent substance at the other terminus.

7. The method according to any one of claims 2 to 5, wherein the amplified product of the detection region of the mutated gene is stained with a nucleic acid stain.

8. The method according to any one of claims 2 to 7, wherein an increase of the amplified product of the detection region of the mutated gene is continuosly detected by an increase of fluorescent intensity.

9. The method according to any one of claims 2 to 8, wherein the mutation is a point mutation.

10. The method according to any one of claims 2 to 8, wherein the mutation is a deletion mutation.

11. The method according to any one of claims 2 to 10, wherein the clamp primer has a chain length of 14 to 18 nucleotides.

12. The method according to claim 1, wherein the gene detection method in step (2) is an invader method, a TaqMan-PCR method, a Scorpion ARMS method, a DNA chip method, a PCR-RFLP method, a PCR-SSCP method, a PCR-HRM method, a gFCS method, or a Southern blotting method.

13. The method according to any one of claims 1 to 12, wherein the mutated gene is a mutated gene of a human epidermal growth factor receptor.
